# EUROPÄISCHE PATENTSCHRIFT

(11) **EP 3 555 039 B1**
(45) Veröffentlichungstag und Bekanntmachung des Hinweises auf die Patenterteilung: **05.08.2020**
(21) Anmeldenummer: 17828680.3
(22) Anmeldetag: 14.12.2017
(51) Int. Cl.: C07C 201/08, C07C 205/06

(54) **VERFAHREN UND ANLAGE ZUR ADIABATISCHEN NITRIERUNG VON AROMATEN**
METHOD AND SYSTEM FOR ADIABATIC NITRATION OF AROMATES
PROCÉDÉ ET INSTALLATION DE NITRATION ADIABATIQUE DE COMPOSÉS AROMATIQUES

(30) Priorität: 03.02.2017 DE 102017000973; 30.03.2017 DE 102017106881; 10.05.2017 DE 102017110084
(43) Veröffentlichungstag der Anmeldung: 23.10.2019
(73) Patentinhaber: Josef Meissner GmbH & Co. KG, 50968 Köln (DE)
(72) Erfinder: PÖHLMANN, Jürgen, 50969 Köln (DE); HERMANN, Dr. Heinrich, 50858 Köln (DE); HÄNDEL, Mirko, 53804 Much (DE); WERNITZ, Sophie, 51067 Köln (DE); FANKEL, Stefan, 53639 Königswinter (DE)
(74) Vertreter: Strehlke, Ingo Kurt
(86) Internationale Anmeldenummer: PCT/EP2017/082770
(87) Internationale Veröffentlichungsnummer: WO 2018/141451

(56) Entgegenhaltungen:
- EP-A1- 2 877 442
- EP-A2- 0 373 966
- DE-A1- 1 468 575

## Beschreibung

Die vorliegende Erfindung betrifft das technische Gebiet der Nitrierung, insbesondere die Herstellung nitrierter organischer aromatischer Verbindungen (synonym nachfolgend auch als "Nitroaromaten", "Nitrierprodukte", "aromatische Nitroprodukte", "aromatische Nitroverbindungen", "nitrierte Produkte" oder dergleichen bezeichnet), vorzugsweise durch adiabatische Nitrierung.

Insbesondere betrifft die vorliegende Erfindung ein Verfahren zur Nitrierung, insbesondere adiabatischen Nitrierung, von nitrierbaren aromatischen organischen Verbindungen (Aromaten) zu den entsprechenden nitrierten aromatischen organischen Verbindungen (Nitroaromaten).

Des Weiteren betrifft die vorliegenden Erfindung eine Produktionsanlage (Nitrieranlage bzw. Anlage) zur Nitrierung, insbesondere adiabatischen Nitrierung, von nitrierbaren aromatischen organischen Verbindungen (Aromaten) zu nitrierten Produkten in Form der entsprechenden nitrierten aromatischen organischen Verbindungen (Nitroaromaten), insbesondere eine Produktionsanlage zur Durchführung des erfindungsgemäßen Verfahrens.

Schließlich betrifft die vorliegende Erfindung die erfindungsgemäße Verwendung von nitrierten aromatischen organischen Verbindungen (Nitroaromaten).

Aromatische Nitroverbindungen (wie z. B. Nitrobenzol (MNB), Mononitrotoluol (MNT), Dinitrotoluol (DNT), Trinitrotoluol (TNT), Nitrochlorbenzol (MNCB) etc.) werden üblicherweise durch Nitrierung entsprechender Ausgangsaromaten (wie z. B. Benzol, Toluol, Xylol, Chlorbenzol, Dichlorbenzolen etc.) hergestellt, insbesondere durch Umsetzung der entsprechenden Ausgangsaromaten mit Salpetersäure in Gegenwart von Schwefelsäure als Katalysator und wasserbindendes Mittel, d. h. also durch Umsetzung der entsprechenden Ausgangsaromaten mit einer Nitriersäure (d. h. einem Salpetersäure/Schwefelsäure-Nitriersäuregemisch, welches zu Beginn der Umsetzung auch als Mischsäure und am Ende der Umsetzung auch als (Nitrier-)Endsäure bezeichnet wird).

Im Stand der Technik wird die Nitrierung der Aromaten mit dem Salpetersäure/ Schwefelsäure-Nitriersäuregemisch bevorzugt als heterogenes flüssig/flüssig-Gemisch aus Organ- und Säurephase durchgeführt, wobei die Umsetzung der zu nitrierenden Aromaten mit der Salpetersäure zum entsprechenden Nitroaromaten in der Säurephase stattfindet. Der zu nitrierende Aromat muss daher zu diesem Zweck zunächst aus der Organphase in die Säurephase überführt werden, um hiermit reagieren zu können. Der in der Säurephase gebildete Nitroaromat wird dann - nach Überschreiten der Löslichkeitsgrenze - als Organphase abgeschieden; diese Organphase besteht zu Beginn der Nitrierung vorwiegend aus dem zu nitrierenden Aromaten und am Ende der Umsetzung, wenn entweder die gesamte Salpetersäure oder zu nitrierender Aromat umgesetzt sind, hauptsächlich aus dem gewünschten Nitroaromaten.

Voraussetzung für eine schnelle und effektive Umsetzung des Aromaten ist, dass stets ausreichend zu nitrierender Aromat aus der Organphase in die Säurephase transferiert wird. Dies wird typischerweise durch Erzeugung einer möglichst großen Austauschfläche zwischen den beiden vorgenannten Phasen erreicht, insbesondere entweder durch das Dispergieren der Organphase in der Säurephase (Öl-in-Wasser- oder O/W-Emulsion) oder umgekehrt der Säurephase in der Organphase (Wasser-in-ÖI- oder W/O-Emulsion). Je größer die Austauschfläche zwischen Organ- und Säurephase ist (d. h. also je kleiner die Tropfengröße der dispergierten Phase), desto größer ist der Umsatz bei der Nitrierung, wie z. B. bei der Nitrierung von Benzol zu Nitrobenzol oder von Toluol zu Mononitrotoluol etc. (wie bei allen sogenannten massentransferkontrollierten Reaktionen).

Wenn beispielsweise die Nitrierung kontinuierlich und isotherm in Rührkesseln bzw. in Rührkesselkaskaden im Gleich- und/oder Gegenstrom durchgeführt wird, liegen in jedem Reaktor konstante Bedingungen, wie z. B. Zusammensetzung der Organphase und der Säurephase, und - damit verbunden - konstante physikalisch-chemisch Bedingungen bzw. Parameter für das zweiphasige Gemisch aus Organphase und Säurephase vor. Die Nitrierung verläuft in jedem Reaktor unter stets gleichen Bedingungen.

Ganz anders dagegen verläuft eine Nitrierung in einem Rührkessel im Chargenbetrieb mit vollständiger Rückvermischung oder in einem Rohrreaktor mit Pfropfenströmung (sogenannter *"plug flow"*) ohne Rückvermischung. In beiden Fällen verändert sich bei Fortschreiten der Nitrierung nicht nur die Zusammensetzung der Organphase und der Säurephase ständig, sondern auch deren physikalisch-chemische Bedingungen bzw. Parameter, wie insbesondere Dichte, Grenzflächenspannung etc. Es ist unter sich ständig ändernden Bedingungen bzw. Parametern viel schwieriger bzw. nahezu unmöglich, über die gesamte Zeit der Umsetzung eine konstant gleiche Austauschfläche für eine kontrollierte Umsetzung zu erzeugen.

Es ist ferner bekannt, dass reine Aromaten, wie z. B. Benzol oder Toluol, in Schwefelsäure oder Nitriersäuregemischen nur schwierig zu dispergieren sind und dass Dispersionen von Aromaten in Schwefelsäure oder Nitriersäuregemischen relativ rasch wieder zerfallen. Wie beispielsweise in der EP 0 373 966 A2 beschrieben, wird bei einmaliger Dispergierung des zu nitrierenden Aromaten (Benzol) in einer Mischsäure wegen zu schneller Koaleszenz der Organphase nur ein Umsatz an Salpetersäure von 55,3 % bzw. an Benzol von 52,5 % erzielt. Es ist daher erforderlich, bei einer Nitrierung, bei welcher zwei Phasen im Nitriergemisch vorliegen (nämlich einerseits eine Organphase aus zu nitrierendem Aromaten und erzeugtem Nitroaromaten und andererseits Nitriersäuregemisch), ständig Mischenergie zuzuführen derart, dass die notwendige Austauschfläche zwischen den beiden Phasen erhalten bleibt, damit bei gegebener Verweilzeit der gewünschte Umsatz erreicht wird.

Vor allem zu Beginn einer Nitrierung (z. B. in einem Rohrreaktor) muss besonders viel Mischenergie zugeführt werden, um eine genügend große Austauschfläche zwischen Organphase und Säurephase zu erzeugen und aufrechtzuerhalten, damit die Nitrierung initiiert wird bzw. anspringt und auch fortschreitet. Geschieht dies nicht, erfolgt durch eine mehr oder weniger schnelle Koaleszenz der dispergierten Phase eine dramatische Verkleinerung der Austauschfläche, verbunden mit einem drastischen Rückgang des Umsatzes des zu nitrierenden Aromaten pro Zeiteinheit.

Wird beispielweise in einem Rohrreaktor nicht durch erneuten Eintrag von Mischenergie in eine einmalig erzeugte Dispersion des zu nitrierenden Aromaten (z. B. Benzol) in der Nitriersäuremischung über die gesamte Länge des Reaktors stets dafür gesorgt (wie z. B. in der EP 1 272 268 A2, der EP 1 291 078 A2 oder der EP 0 708 076 A2 beschrieben), dass die einsetzende Koaleszenz der in der Nitriersäure dispergierten Organphase durch Zufuhr von zusätzlicher Mischenergie verhindert wird, bricht die Nitrierung zusammen - was daran erkennbar ist, dass keine Nitrierwärme mehr freigesetzt wird, obwohl in der Nitriersäure noch Salpetersäure und in der Organphase noch zu nitrierender Aromat vorliegt.

Entsprechendes gilt für den Beginn der Umsetzung: Ist die zu Beginn der Umsetzung erzeugte Tropfengröße nicht klein genug und ist damit die Austauschfläche zu klein, läuft z. B. die massentransferkontrollierte Umsetzung von Benzol oder Toluol zu Nitrobenzol oder Mononitritoluol nur langsam ab - was daran erkennbar ist, dass kein bzw. nur ein geringer Temperaturanstieg im Reaktionsgemisch beobachtet wird, da die Umsetzung nicht startet. Umgekehrt erfolgt, wenn die Tropfengroße der dispergierten Phase ausreichend klein und damit die Austauschfläche groß ist, unter sonst gleichen Bedingungen eine rasche Umsetzung des zu nitrierenden Aromaten - was daran erkennbar ist, dass ein rascher Temperaturanstieg im Reaktionsgemisch und, damit verbunden, ein gewünschter hoher Umsatz an zu nitrierendem Aromat zum entsprechenden Nitroaromaten zu beobachten ist.

Vor allem bei adiabatischen Umsetzungen (z. B. bei der adiabatischen Nitrierung von Benzol zu Nitrobenzol) hängt der Umsatz und die dafür benötigte Zeit - neben der Austauschfläche zwischen den beiden Phasen und damit der Tropfengröße der dispergierten Phase - noch von weiteren allgemein bekannten Parametern ab, wie z. B. der Konzentration der Schwefelsäure und Salpetersäure in der Nitriersäure (zu Beginn der Umsetzung als Mischsäure und am Ende als Endsäure bezeichnet), der Starttemperatur (vgl. z. B. EP 2 168 942 A1), dem Phasenverhältnis zwischen Organ- und Säurephase und damit verbunden der Endtemperatur etc., ab.

Der Umsatz bei einer adiabatische Nitrierung (z. B. von Benzol zu Nitrobenzol) im Rohrreaktor wird - ausgehend von einer definierten Starttemperatur - durch den Anstieg der Temperatur im Nitriergemisch infolge der freigesetzten Nitrierwärme, charakterisiert (vgl. z. B. EP 2 168 942 A1 und EP 1 272 268 A2). Die für ein bestimmtes Nitriergemisch ermittelte Temperaturdifferenz (auch Delta T bzw. ΔT genannt) kann direkt, insbesondere linear, mit dem Umsatz an Salpetersäure korreliert werden, wie z. B. in der EP 2 168 942 A1 beschrieben.

Um bei vorgegebener Verweilzeit in einem Rohrreaktor den größtmöglichen Umsatz zu erreichen (z. B. mehr als 98 % der eingesetzten Salpetersäure), ist neben einer optimalen Dispergierung des zu nitrierenden Aromaten in der Mischsäure auch eine geeignete Starttemperatur nötig, damit die Reaktion initiiert wird bzw. anspringt, d. h. dass nach Vermischung der Edukte eine Umsetzung derart erfolgt, dass ein steiler, insbesondere gleichmäßiger, vorzugsweise exponentieller Temperaturanstieg im Nitriergemisch beobachtet wird, so dass z. B. in den ersten 13 Vol.-% des Reaktionsraumes eines Rohrreaktors mindestens 60 % der eingesetzten Salpetersäure umgesetzt werden (vgl. z. B. EP 2 168 942 A1). Dies wird beispielsweise durch eine spezielle Anordnung der Dispergierelemente für die notwendige Redispergierung der zu Beginn schnell koaleszierenden Organphase im Rohrreaktor erreicht (vgl. z. B. EP 1 272 269 A1).

Die Starttemperatur kann beispielsweise im Bereich von 50 bis 120 °C gewählt werden. Durch Zusammenmischen von verschieden temperierten Eduktströme (d. h. Schwefelsäure, Salpetersäure und zu nitrierender Aromat, wie z. B. Benzol, vgl. beispielsweise EP 0 436 443 A2 oder EP 1 272 269 A1) bildet sich eine Mischtemperatur, wobei der Hauptbeitrag zur Starttemperatur aus der im großen Überschuss vorliegenden Schwefelsäure stammt.

Mit der Starttemperatur wird - bei gegebener Verweilzeit - aber nicht nur der Umsatz gesteuert, sondern auch die Bildung der für eine adiabatische Nitrierung beispielsweise von Benzol zu Nitrobenzol typischen Nebenprodukte, wie insbesondere die Menge der Di- und Trinitrophenole (Pikrinsäure) und von Dinitrobenzol (DNB).

Mit Starttemperaturen von 80 bis 120 °C (vgl. z. B. US 4 091 042 A), bevorzugt größer 97 °C und besonders bevorzugt von 100 bis 120 °C (vgl. z.B. EP 0 436 443 A2 oder EP 2 168 942 A1), ist es möglich, mit Verweilzeiten im Reaktionsrohr von weniger als 2 Minuten (z. B. maximal 25 Sekunden, vgl. z. B. EP 0 436 443 A2) Umsätze an Salpetersäure von mindestens 99 % zu erreichen.

Mit tieferen Starttemperaturen werden hingegen wesentlich längere Verweilzeiten benötigt. Mit einer Starttemperatur beispielsweise von ca. 80 °C wird mit Anlagen nach dem Stand der Technik (Rohrreaktor) eine Verweilzeit von 300 Sekunden als notwendig beschrieben, um einen vollständigen Umsatz der Salpetersäure zu erreichen (vgl. z. B. US 8 692 035 B2 oder WO 2010/051616 A1).

Im Vergleich zu Anlagen nach dem Stand der Technik, in welchen mit Starttemperaturen um 97 bis 110 °C gearbeitet wird, werden daher für niedrigere Starttemperaturen wesentlich größere Nitrierreaktoren benötigt, welche allerdings deutlich kostenintensiver sind, da diese üblicherweise aus emailliertem Stahl ausgebildet sind.

Bei einer Anlagenleistung von z. B. 20 Tonnen Nitrobenzol (NB) pro Stunde (d. h. 20 t NB/h) würde ein Rohrreaktor mit einem Durchmesser von 250 mm bei einer Fließgeschwindigkeit des Nitriergemisches von 1,25 m/s und einer Verweilzeit von mindestens 300 s um den Faktor 2,5 länger sein (d. h. ca. 375 m) als ein Standardreaktor nach dem Stand der Technik von 150 m Länge bei einer Verweilzeit von 120 s und ansonsten gleichen Bedingungen (d. h. gleiche Mischsäurezusammensetzung, gleiches Phasenverhältnis etc.).

Ein weiteres Ziel einer Optimierung von Anlagen zur adiabatischen Nitrierung von Aromaten, insbesondere Benzol, besteht darin, die Menge der Nebenprodukte im Nitrobenzol zu minimieren. Wie bereits in der EP 0 436 443 A2 beschrieben, nimmt die Bildung von Di- und Trinitrophenolen mit steigender Endtemperatur des Nitriergemisches rasch zu. Auch aus diesen Gründen sollte die Endtemperatur im Nitriergemisch 135 bis 145 °C nicht überschreiten. Der Gehalt an Nitrophenolen im Rohnitrobenzol (Roh-NB) bewegt sich dann im Bereich von 2.000 bis 3.000 ppm. Der Gehalt an Dinitrobenzol (DNB) bewegt sich bei diesen Endtemperaturen im Bereich von 200 bis 250 ppm. Die Entfernung dieser Nitrophenole aus dem Rohnitrobenzol und ihre Vernichtung im Abwasser, wie z. B. mittels einer Thermolyse (wie in der EP 0 953 546 A2 und der EP 0 005 203 A2 beschrieben), ist aufwendig und teuer.

Durch Absenken der Starttemperatur und damit auch der Endtemperatur kann die Bildung von Nebenprodukten stark reduziert werden. Jede Absenkung der Starttemperatur um 20 bis 25 °C führt zu einer Halbierung des Nitrophenolgehalts im Rohnitrobenzol. Die Absenkung der Starttemperatur von ca. 110 °C auf ca. 80 °C führt zu einer Reduzierung des Nitrophenolgehaltes um ca. 50 %, d. h. auf etwa 1.500 ppm und weniger (z. B. 1.000 ppm), im Vergleich zu den Verhältnissen, wie sie beispielsweise in der EP 0 436 443 A2 beschrieben sind, besonders bevorzugt bis auf einen Wert um 1000 ppm. Der Gehalt an Dinitrobenzol (DNB) fällt analog auf ca. 100 ppm (vgl. hierzu beispielsweise auch US 8 692 035 B2 oder WO 2010/051616 A1).

Durch das Phasenverhältnis zwischen Säurephase und Organphase kann - bei gegebener Salpetersäurekonzentration in der Mischsäure - der Temperaturanstieg im Nitriergemisch zwischen Starttemperatur und Endtemperatur gesteuert werden. Bei konstantem Phasenverhältnis und gleichbleibender Schwefelsäurekonzentration und Starttemperatur in der Ausgangsmischsäure steigt mit zunehmendem Gehalt an Salpetersäure in der Mischsäure und bei gleichem Umsatz die Endtemperatur im Nitriergemisch und umgekehrt.

Wie bereits in der EP 0 771 783 A1 beschrieben, ist es für eine hohe Selektivität vorteilhaft, dass zu Beginn der Umsetzung im Rohrreaktor durch eine optimale Durchmischung der Phasen ein hoher Anfangsumsatz erreicht wird. Nach dem Stand der Technik werden verschiedene Maßnahmen beschrieben, wie eine möglichst optimale Dispergierung des zu nitrierenden Aromaten in der Ausgangsmischsäure zu Beginn der Umsetzung und eine Redispergierung zu erreichen ist (vgl. z. B. EP 0 373 966 A2, EP 0 489 211 A1, EP 0 771 783 A1, EP 0 779 270 A1, EP 1 272 269 A1, EP 1 291 078 A2 und EP 2 168 942 A1).

Eine optimale Dispergierung des zu nitrierenden Aromaten (z. B. Benzol) in der Nitriersäure besonders zu Beginn der Nitrierung, um die Umsetzung zu starten, ist Voraussetzung für einen hohen Umsatz (vgl. EP 1 272 269 A1 oder EP 2 168 942 A1). Wie diesbezüglich in der US 9 284 256 B2 und der EP 2 877 442 A1 beschrieben, kann die Zumischung von mehr als 4 % an aliphatischen Kohlenwasserstoffen zu dem zu nitrierenden Benzol dazu führen, dass die Nitrierung nicht startet. d. h. dass kein merkbarer Temperaturanstieg im Nitriergemisch nach Zusammenmischen der Edukte und der ersten Dispergierung beobachtet wird, während unter sonst gleichen Bedingungen mit einem Benzol mit einem Gehalt an weniger als 0,1 % an Aliphaten die Nitrierung (wie in der EP 1 272 269 A1 oder der EP 2 168 942 A1 beschrieben) mit einem steilen Temperaturanstieg in den ersten 13 Vol.-% des Rohrreaktors startet und wie vorgesehen abläuft.

Im Stand der Technik hat es nicht an Versuchen gefehlt, eine verbesserte Dispergierung des zu nitrierenden Aromaten in der Nitriersäure zu erreichen. Eine aus dem Stand der Technik bekannte Maßnahme, um dies zu erreichen, ist beispielsweise ein großes Verhältnis von Säurephase zu Organphase, wodurch die Dispergierbarkeit der Organphase verbessert und die Koaleszenz reduziert werden soll, wie beispielsweise in der EP 0 436 443 A2 und der US 8 692 035 B2 beschrieben. Eine weitere, aus dem Stand der Technik bekannte Maßnahme, wie in der EP 1 272 268 A2 und der EP 2 168 942 A1 ausgeführt, besteht darin, die Nitrierung in einem Rohrreaktor mit Misch- bzw. Dispergierelementen durchzuführen und dabei durch eine nichtgleichmäßige Anordnung bzw. Verteilung der Misch- bzw. Dispergierelemente über die gesamte Länge des Rohrreaktors zu erreichen, dass zu Beginn der Umsetzung ein gleichmäßiger, insbesondere exponentieller, vorzugsweise S-förmiger Temperaturanstieg erfolgt und ein möglichst höher Umsatz im vorderen Abschnitt des Rohrreaktors erreicht wird. Die aus dem Stand der Technik bekannten Maßnahmen sind aber nicht ausreichend, um stets eine optimale Dispergierung des zu nitrierenden Aromaten in der Nitriersäure zu erreichen und die mit einer unzureichenden Dispergierung verbundenen und zuvor geschilderten Probleme und Nachteile auszugleichen.

Weiterhin betrifft die DE 1 468 575 A1 ein Verfahren zum Herstellen der Mononitroverbindungen von Benzol, Toluol oder Chlorbenzol durch isothermes Nitrieren mittels Nitriersäure (Salpetersäure/Schwefelsäure-Gemisch), sowie eine Vorrichtung zur Durchführung dieses Verfahrens.

Darüber hinaus betrifft die EP 0 373 966 A2 ein Verfahren zur Mononitrierung einer nitrierbaren organischen Verbindung mit einer Mischsäure, wobei einer der Reaktanten mittels einer Zerstäuberdüse oder einer ähnlichen Öffnung in Form von ultrafeinen Tröpfchen in den anderen Reaktanten eingebracht wird.

Die EP 2 877 442 A1 (bzw. die WO 2014/016292 A1) betrifft ein Verfahren zur Herstellung von Nitrobenzol durch adiabatische Nitrierung von Benzol mit Mischungen aus Schwefel- und Salpetersäure unter Einsatz eines stöchiometrischen Überschusses an Benzol, wobei der Gehalt an aliphatischen organischen Verbindungen im Einsatzbenzol während der Anfahrtszeit der Produktionsanlage stets kleiner als 1,5 Masse-%, bezogen auf die Gesamtmasse des Einsatzbenzols, gehalten wird, wobei dies dadurch erreicht wird, dass entweder während der Anfahrtszeit das Einsatzbenzol aus rezykliertem nicht umgesetzten Benzol (Rückbenzol) und der Reaktion neu zugeführtem Benzol (Frischbenzol), abhängig von der Reinheit beider Ströme, in entsprechenden Mengenverhältnissen gemischt wird, oder indem während der Anfahrtszeit auf die Rezyklierung nicht umgesetzten Benzols komplett verzichtet wird, das Einsatzbenzol also nur aus der Reaktion neu zugeführtem Benzol besteht.

Eine Aufgabe der vorliegenden Erfindung ist es daher, ein Verfahren und eine entsprechende, d. h. zur Durchführung dieses Verfahrens geeignete Produktionsanlage (Nitrieranlage bzw. Anlage) zur Nitrierung, insbesondere adiabatischen Nitrierung, von nitrierbaren aromatischen organischen Verbindungen (Aromaten) bereitzustellen, wobei die zuvor geschilderten Nachteile und Unzulänglichkeiten des Standes der Technik zumindest weitgehend vermieden oder aber wenigstens abgeschwächt werden sollen.

Insbesondere ist eine Aufgabe der vorliegenden Erfindung darin zu sehen, ein Verfahren und eine entsprechende, zur Durchführung dieses Verfahrens geeignete Produktionsanlage (Nitrieranlage bzw. Anlage) zur Nitrierung, insbesondere adiabatischen Nitrierung, von nitrierbaren aromatischen organischen Verbindungen (Aromaten) bereitzustellen, mit welchem bzw. welcher die nitrierbaren aromatischen organischen Verbindungen in technisch effizienter wie sicherer und einfacher Art und Weise zu den entsprechenden nitrierten aromatischen organischen Verbindungen (Nitroaromaten) umgesetzt bzw. reagiert werden können.

Weiterhin ist eine Aufgabe der vorliegenden Erfindung darin zu sehen, ein Verfahren und eine entsprechende, zur Durchführung dieses Verfahrens geeignete Produktionsanlage (Nitrieranlage bzw. Anlage) zur Nitrierung, insbesondere adiabatischen Nitrierung, von nitrierbaren aromatischen organischen Verbindungen (Aromaten) bereitzustellen, wobei im Rahmen der Nitrierung die Dispergierung der zu nitrierenden Aromaten in dem Nitriersäuregemisch, insbesondere zu Beginn Reaktion, vorzugsweise bereits nach dem Zusammenmischen der Edukte, verbessert wird. Insbesondere soll dabei die Dispergierung von Organphase und Säurephase auch unter ungünstigen Bedingungen (wie z. B. bei Gegenwart von Verunreinigungen, wie z. B. einem erhöhten Gehalt an Aliphaten im zu nitrierenden Aromaten, bei einer zu tiefen Starttemperatur oder bei einem geringen Eintrag von Dispergierenergie) derart verbessert sein, dass die Nitrierung noch effizient durchführbar ist, insbesondere das Nitriergemisch in der vorgegebenen Reaktions- bzw. Verweilzeit im Reaktor zu mindestens 98 % umgesetzt werden kann. Schließlich ist eine Aufgabe der vorliegenden Erfindung auch darin zu sehen, ein Verfahren und eine entsprechende, zur Durchführung dieses Verfahrens geeignete Produktionsanlage (Nitrieranlage bzw. Anlage) zur Nitrierung, insbesondere adiabatischen Nitrierung, von nitrierbaren aromatischen organischen Verbindungen (Aromaten) bereitzustellen, wobei im Rahmen der Nitrierung eine verbesserte Dispergierung bereits nach Inkontaktbringen (z. B. Zusammenmischen) der Reaktanden, insbesondere von Salpetersäure enthaltender Nitriersäurephase einerseits und von die zu nitrierenden Aromaten enthaltender Organphase andererseits, vorzugsweise unter Vermeidung einer schnellen Koaleszenz der zu nitrierenden Aromaten in der Nitriersäure, erreicht werden kann, vorzugsweise mit dem Ziel, die Nitrierreaktion auch mit im Vergleich zum Stand der Technik geringeren Starttemperaturen zu starten (und zwar, ohne dass längere Reaktions- oder Verweilzeiten als bei Verfahren nach dem Stand der Technik mit höheren Starttemperaturen, aber ansonsten gleichen Bedingungen benötigt werden).

Die zuvor geschilderte Aufgabenstellung wird erfindungsgemäß - gemäß einem **ersten** Erfindungsaspekt - durch ein Verfahren nach Anspruch 1 gelöst; weitere, vorteilhafte Weiterbildungen und Ausgestaltungen des erfindungsgemäßen Verfahrens sind Gegenstand der diesbezüglichen Unteransprüche.

Weiterer Gegenstand der vorliegenden Erfindung - gemäß einem **zweiten** Erfindungsaspekt - ist eine Produktionsanlage (Nitrieranlage bzw. Anlage) nach dem betreffenden unabhängigen Produktionsanlage-Anspruch; weitere, vorteilhafte Weiterbildungen und Ausgestaltungen dieses Erfindungsaspekts sind Gegenstand der diesbezüglichen Unteransprüche (Produktionsanlage-Unteransprüche).

Schließlich ist Gegenstand der vorliegenden Erfindung - gemäß einem **dritten** Erfindungsaspekt - die erfindungsgemäße Verwendung nach den betreffenden unabhängigen Verwendungsansprüchen; weitere, vorteilhafte Ausgestaltungen und Weiterbildungen dieses Erfindungsaspekts sind Gegenstand der diesbezüglichen Unteransprüche (Verwendungsunteransprüche).

Es versteht sich von selbst, dass Ausgestaltungen, Ausführungsformen, Vorteile oder dergleichen, welche nachfolgend -zu Zwecken der Vermeidung von unnötigen Wiederholungen - nur zu einem Erfindungsaspekt ausgeführt werden, selbstverständlich auch in Bezug auf alle übrigen Erfindungsaspekte entsprechend gelten.

Weiterhin versteht es sich von selbst, dass bei der nachfolgenden Angabe von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungs- bzw. Analysemethoden ermittelt bzw. bestimmt werden können.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist somit ein Verfahren zur adiabatischen Nitrierung von nitrierbaren aromatischen organischen Verbindungen (Aromaten) zu den entsprechenden nitrierten aromatischen organischen Verbindungen (Nitroaromaten),
wobei nitrierbare aromatische organische Verbindungen (Aromaten) in einer Nitrierreaktion mit einem Salpetersäure/Schwefelsäure-Nitriersäuregemisch zu den entsprechenden nitrierten aromatischen organischen Verbindungen (Nitroaromaten) umgesetzt werden,
wobei der Ausgangsreaktionsmischung, welche die nitrierbaren aromatischen organischen Verbindungen (Aromaten) und das Salpetersäure/Schwefelsäure-Nitriersäuregemisch umfasst, entsprechende nitrierte aromatische organische Verbindungen (Nitroaromaten) zugesetzt werden und die Umsetzung und/oder Nitrierreaktion in Gegenwart der nitrierten aromatischen organischen Verbindungen (Nitroaromaten) gestartet und/oder durchgeführt wird; und
wobei die erhaltenen nitrierten aromatischen organischen Verbindungen (Nitroaromaten) teilweise in die Nitrierreaktion zurückgeführt werden und die nachfolgende Umsetzung und/oder Nitrierreaktion in Gegenwart der nitrierten aromatischen organischen Verbindungen (Nitroaromaten) gestartet und/oder durchgeführt wird;
wobei die Umsetzung und/oder Nitrierreaktion unter adiabatischen Reaktionsbedingungen durchgeführt wird,
wobei die Umsetzung und/oder Nitrierreaktion in einem Rohrreaktor durchgeführt wird und
wobei die für die Umsetzung und/oder Nitrierreaktion zugesetzte und/oder zurückgeführte Menge an nitrierten aromatischen organischen Verbindungen (Nitroaromaten) derart ausgewählt wird, dass die Menge an zugesetzten und/oder zurückgeführten nitrierten aromatischen organischen Verbindungen (Nitroaromaten) eine Absenkung der Grenzflächenspannung zwischen Organphase und Säurephase bewirkt und eine verbesserte Dispergierbarkeit von Organphase und Säurephase bewirkt.

Der Begriff der entsprechenden nitrierten aromatischen organischen Verbindungen (Nitroaromaten), wie er erfindungsgemäß verwendet wird, bezeichnet im Rahmen der vorliegenden Erfindung insbesondere diejenigen Nitroaromaten, welche sich durch Nitrierung, vorzugsweise Mononitrierung, der eingesetzten Ausgangsaromaten herstellen lassen. So ist beispielsweise Mononitrobenzol (MNB) die entsprechende nitrierte aromatische organische Verbindung (Nitroaromat) zu Benzol, Mononitrotoluol (MNT) zu Toluol, Mononitrochlorbenzol zu Chlorbenzol etc.

Das erfindungsgemäße Verfahren - ebenso wie die nachfolgend noch im Detail beschriebene erfindungsgemäße (Produktions-)Anlage zur Durchführung des erfindungsgemäßen Verfahrens - ist mit zahlreichen Besonderheiten und Vorteilen verbunden, auf welche nachfolgend eingegangen werden soll:
Die Anmelderin hat im Rahmen der vorliegenden Erfindung überraschenderweise herausgefunden, dass der Zusatz von nitriertem Produkt zu der Ausgangsreaktionsmischung zu einer Erniedrigung der Grenzflächenspannung zwischen Organphase einerseits und saurer wässriger Phase bzw. Säurephase andererseits führt (wobei die Organphase die zu nitrierenden Ausgangsaromaten und die zugesetzten nitrierten Produkte sowie gegebenenfalls Nitriernebenprodukte umfasst und wobei die saure wässrige Phase bzw. die Säurephase in der Ausgangsreaktionsmischung zu Beginn der Umsetzung Schwefelsäure und nach Zusatz der Salpetersäure die Nitriersäure bzw. Mischsäure und am Ende der Umsetzung die sogenannte wässrige Nitrierendsäure bzw. das sogenannte wässrige Nitrierendsäuregemisch und gegebenenfalls hierin gelöste Anteile von zugesetztem und/oder bei der Nitrierung gebildetem Nitroaromaten umfasst). Hierdurch wird also eine signifikant verbesserte Dispergierbarkeit bzw. Emulgierbarkeit zwischen Organphase einerseits und wässriger saurer Phase bzw. Säurephase andererseits erreicht, d. h. das nitrierte Endprodukt bzw. der nitrierte Aromat fungiert somit in der Ausgangsreaktionsmischung als Dispergator (Dispergiermittel) bzw. Emulgator. Dabei handelt es sich sozusagen um einen systemeigenen bzw. systeminhärenten Dispergator bzw. Emulgator, da keinerlei reaktionsfremde Substanzen zum Einsatz kommen, welche die Reaktionsprodukte verunreinigen können.

Insgesamt wird durch den Zusatz von nitriertem Produkt zu der Ausgangsreaktionsmischung eine verbesserte, insbesondere innige Vermischung und Dispergierung der beiden Phasen (d. h. Organphase und Säurephase) erreicht, so dass insgesamt ein verbesserter und schnellerer Austausch zwischen den beiden Phasen stattfindet. Hierdurch bedingt, erfolgt auch eine schnellere Umsetzung bzw. schnellere Nitrierreaktion, insbesondere mit verbesserten Ausbeuten, insbesondere verbesserten Raum/Zeit-Ausbeuten.

Auch werden im Rahmen der erfindungsgemäßen Reaktionsführung signifikant weniger Nebenprodukte gebildet. Insgesamt kann durch den Zusatz von nitriertem Produkt zu der Ausgangsreaktionsmischung die Reaktions- oder Verfahrensführung bzw. die Nitrierreaktion besser gesteuert werden.

Neben den verkürzten Reaktionsdauern und den schnelleren Umsetzungen mit verbesserten Ausbeuten und geringerer Nebenproduktbildung kann im Rahmen der erfindungsgemäßen Verfahrensführung - im Vergleich zu herkömmlichen Nitrierverfahren mit ansonsten gleichen Bedingungen - auch die Starttemperatur für das Anfahren bzw. die Initiierung der Reaktion signifikant abgesenkt werden, d. h. im Rahmen der erfindungsgemäßen Verfahrensführung können signifikant geringere (Reaktions-)Starttemperaturen für die Nitrierreaktion verwendet werden, wie nachfolgend noch ausgeführt wird.

Insgesamt wird daher im Rahmen der vorliegenden Erfindung ein verbessertes adiabatisches Nitrierverfahren für nitrierbare aromatische organische Verbindungen bereitgestellt, welches sich durch eine insgesamt verbesserte Effizienz, insbesondere eine verbesserte technische Effizienz sowie eine verbesserte Energieeffizienz, und eine insgesamt verbesserte Verfahrensökonomie und zudem eine verbesserte Handhabbarkeit auszeichnet.

Im Rahmen der vorliegenden Erfindung lässt sich bei einer adiabatischen Reaktionsführung der Nitrierung (z. B. adiabatische Nitrierung von Benzol zu Mononitrobenzol in einem Rohrreaktor) die Dispergierung des zu nitrierenden Aromaten in dem Nitriersäuregemisch, insbesondere bereits zu Beginn der Reaktion, vorzugsweise bereits nach dem Inkontaktbringen (z. B. Zusammenmischen) der Edukte, derart verbessern, dass selbst unter ungünstigen Bedingungen (wie z. B. bei Gegenwart von Verunreinigungen, wie z. B. einem erhöhten Gehalt an Aliphaten im zu nitrierenden Aromaten oder bei einer zu tiefen Starttemperatur oder bei einem geringen Eintrag von Dispergierenergie) die Nitrierung noch effizient durchführbar ist, insbesondere das Nitriergemisch in der vorgegebenen Reaktions- bzw. Verweilzeit im Reaktor zu mindestens 98 %, insbesondere zu mindestens 99 %, vorzugsweise zu mindestens 99,5 %, jeweils bezogen auf den Salpetersäureumsatz im Nitriersäuregemisch, umgesetzt werden kann.

Durch das erfindungsgemäße Verfahren kann im Rahmen der Nitrierung eine verbesserte Dispergierung bereits nach Inkontaktbringen (z. B. Zusammenmischen) der Reaktanden, insbesondere von Salpetersäure enthaltender Nitriersäurephase einerseits und von die zu nitrierenden Aromaten enthaltender Organphase andererseits, vorzugsweise unter Vermeidung einer schnellen Koaleszenz der zu nitrierenden Aromaten in der Nitriersäure, erreicht werden, vorzugsweise mit der Zielsetzung, die Nitrierreaktion auch mit im Vergleich zum Stand der Technik geringeren Starttemperaturen zu starten (und zwar, ohne dass längere Reaktions- oder Verweilzeiten als bei Verfahren nach dem Stand der Technik mit höheren Starttemperaturen, aber ansonsten gleichen Bedingungen benötigt werden). Insbesondere lässt sich durch die erfindungsgemäße Verfahrensführung erreichen, dass auch bei (Reaktions-)Starttemperaturen unterhalb von 100 °C, bevorzugt unterhalb von 95 °C, besonders bevorzugt unterhalb von 90 °C, die Nitrierung derart initiiert bzw. gestartet werden kann, dass keine längeren Reaktions- bzw. Verweilzeiten als bei Verfahren nach dem Stand der Technik mit höheren Starttemperaturen, aber ansonsten gleichen Bedingungen benötigt werden.

Durch die vorliegende Erfindung können also nitrierbare aromatische organische Verbindungen (d. h. Aromaten) in technisch effizienter wie sicherer und einfacher Art und Weise zu den entsprechenden nitrierten aromatischen organischen Verbindungen (Nitroaromaten) umgesetzt bzw. reagiert werden.

Im Rahmen der vorliegenden Erfindung wurde also vollkommen überraschend gefunden, dass durch Zumischen von nitriertem Produkt (z. B. Nitrobenzol im Fall der Nitrierung von Benzol) zu der Ausgangsreaktionsmischung eine signifikant verbesserte Erstdispergierung des zu nitrierenden Aromaten (insbesondere charakterisiert durch einen steileren Temperaturanstieg im Nitriergemisch nach der die Nitrierung auslösenden Erstdispergierung) sowie eine reduzierte Neigung zur Koaleszenz dieser Dispersion beobachtet wird.

Gegenstand der Erfindung ist somit mit anderen Worten insbesondere ein insgesamt verbessertes, adiabatisches Verfahren zur Gewinnung von Nitroaromaten (wie z. B. Nitrobenzol, Nitrotoluol, Nitrochlorbenzol etc.) durch eine adiabatische Umsetzung der entsprechenden Ausgangsaromaten (wie z. B. Benzol, Toluol, Chlorbenzol etc.) mit Salpetersäure in Gegenwart von Schwefelsäure als wasserbindendem Mittel und Katalysator in einem Rohrreaktor und bevorzugt unter Einsatz eines stöchiometrischem Überschusses an zu nitrierendem Aromaten, wobei dem Reaktionsansatz bzw. der Ausgangsreaktionsmischung ein Anteil des entsprechenden Nitroaromaten (z. B. Nitrobenzol im Fall der Nitrierung von Benzol) zugesetzt bzw. zugemischt wird.

Wie nachfolgend noch im Detail geschildert, ist das erfindungsgemäße Verfahren dabei vollkommen flexibel durchführbar: So kann der Anteil an zuzusetzendem bzw. zuzumischendem Nitroaromaten (z. B. Nitrobenzol im Fall der Nitrierung von Benzol) beispielsweise der für die Nitrierung benötigen rezyklierten oder frischen Schwefelsäure und/oder dem zu nitrierenden Aromaten vor dem ersten gemeinsamen Dispergieren der weiteren Edukte (d. h. Schwefelsäure und Salpetersäure) und/oder nur einem Teil des zu nitrierenden Aromaten vor dem ersten gemeinsamen Dispergieren der weiteren Edukte (d. h. Schwefelsäure und Salpetersäure sowie restlicher Teil des zu nitrierenden Aromaten) etc. zugegeben werden, wobei auch Kombinationen dieser Varianten möglich sind. Grundsätzlich kommt es nur darauf an, dass zu Beginn der Nitrierreaktion ausreichende Mengen an Nitroaromat in der Ausgangsreaktionsmischung vorhanden sind, um eine effiziente Dispergierung von Organ- und Säurephase zu gewährleisten.

Nachfolgend werden besondere, vorteilhafte oder bevorzugte Ausführungsformen des erfindungsgemäßen Verfahrens beschrieben:
Wie zuvor beschrieben, wird im Rahmen des erfindungsgemäßen Verfahrens die Umsetzung und/oder Nitrierreaktion unter adiabatischen Reaktionsbedingungen durchgeführt.

Gemäß einer besonderen Ausführungsform der vorliegenden Erfindung wird weiterhin die Umsetzung und/oder Nitrierreaktion insbesondere als Mononitrierung durchgeführt.

Gemäß einer weiteren besonderen Ausführungsform der vorliegenden Erfindung wird üblicherweise derart vorgegangen, dass die Ausgangsreaktionsmischung und die nitrierten aromatischen organischen Verbindungen (Nitroaromaten) unter den gewählten Reaktionsbedingungen als flüssig/flüssig-Gemisch aus Organphase einerseits und Säurephase, insbesondere saurer wässriger Phase, andererseits vorliegen. Insbesondere kann dabei die Organphase nitrierbare aromatische organische Verbindungen (Aromaten) und nitrierte aromatische organische Verbindungen (Nitroaromaten) umfassen und/oder insbesondere kann dabei die Säurephase (d. h. insbesondere die saure wässrige Phase) Salpetersäure, Schwefelsäure und gegebenenfalls Wasser (sowie gegebenenfalls hierin gelöste Anteile von zugesetztem und/oder bei der Nitrierung gebildetem Nitroaromaten) umfassen.

Im Rahmen des erfindungsgemäßen Verfahrens bilden die nitrierbaren aromatischen organischen Verbindungen (Aromaten) und das Salpetersäure/ Schwefelsäure-Nitriersäuregemisch sowie die (zugesetzten oder zurückgeführten) nitrierten aromatischen organischen Verbindungen (Nitroaromaten) das (Anfangs-)-Nitriergemisch (d. h. das anfängliche Nitriergemisch bzw. das zu Beginn der Umsetzung und/oder Nitierreaktion vorliegende Nitriergemisch); mit anderen Worten also umfasst das Nitriergemisch Nitriergemisch (d. h. das anfängliche Nitriergemisch bzw. das zu Beginn der Umsetzung und/oder Nitierreaktion vorliegende Nitriergemisch) die Ausgangsreaktionsmischung, welche die nitrierbaren aromatischen organischen Verbindungen (Aromaten) und das Salpetersäure/ Schwefelsäure-Nitriersäuregemisch sowie die nitrierten aromatischen organischen Verbindungen (Nitroaromaten) umfasst. Dagegen umfasst das nach Umsetzung und/oder am Ende der Nitrierreaktion erhaltene bzw. resultierende Nitriergemisch zumindest im Wesentlichen Nitrierendsäure und nitrierte aromatische organische Verbindungen (Nitroaromaten) sowie gegebenenfalls geringe Mengen an nichtumgesetzten nitrierbaren aromatischen organischen Verbindungen (Aromaten) (neben gegebenenfalls vorhandenen Verunreinigungen und Nebenprodukten).

Gemäß der vorliegenden Erfindung lassen sich grundsätzlich nahezu beliebige nitrierbare aromatische organische Verbindungen (Aromaten) nitrieren.

Gemäß einer besonderen Ausführungsform der vorliegenden Erfindung können die nitrierbaren aromatischen organischen Verbindungen (Aromaten) unter den gewählten Reaktionsbedingungen flüssig sein. Insbesondere können die nitrierbaren aromatischen organischen Verbindungen (Aromaten) unter Standarddruck (1,01325 bar) und bei einer Temperatur ab 70 °C, insbesondere ab 50 °C, vorzugsweise ab 25 °C, besonders bevorzugt ab 10 °C, im flüssigen Aggregatzustand vorliegen. Dies ermöglicht eine effiziente Reaktionsführung.

Insbesondere können die nitrierbaren aromatischen organischen Verbindungen (Aromaten) ausgewählt sein aus gegebenenfalls halogenierten ein- oder mehrkernigen organischen Aromaten.

Gemäß einer besonderen Ausführungsform der vorliegenden Erfindung können die für die Nitrierung eingesetzten nitrierbaren aromatischen organischen Verbindungen (Aromaten) insbesondere ausgewählt sein aus der Gruppe von Benzol, Mononitrobenzol (MNB), halogenierten Benzolen, insbesondere Mono- und Dichlorbenzolen, mononitrierten halogenierten Benzolen, Toluol, Mononitrotoluol (MNT), Dinitrotoluolen (DNT) und Xylolen sowie deren Mischungen und Kombinationen. Besonders bevorzugt ist Benzol.

Gemäß der vorliegenden Erfindung lassen sich grundsätzlich nahezu beliebige nitrierte aromatische organische Verbindungen (Nitroaromaten) herstellen.

Was die hergestellten nitrierten aromatischen organischen Verbindungen (Nitroaromaten) anbelangt, so können diese unter den gewählten Reaktionsbedingungen insbesondere flüssig sein. Insbesondere können die nitrierten aromatischen organischen Verbindungen (Nitroaromaten) unter Standarddruck (1,01325 bar) und bei einer Temperatur ab 70 °C, insbesondere ab 50 °C, vorzugsweise ab 25 °C, besonders bevorzugt ab 10 °C, im flüssigen Aggregatzustand vorliegen. Hierdurch wird ein effizienter Verfahrensablauf gewährleistet.

Insbesondere können die hergestellten nitrierten aromatischen organischen Verbindungen (Nitroaromaten) ausgewählt sein aus gegebenenfalls halogenierten ein- oder mehrkernigen mono-, di- oder trinitrierten organischen Aromaten.

Gemäß einer besonderen Ausführungsform der vorliegenden Erfindung können die hergestellten nitrierten aromatischen organischen Verbindungen (Nitroaromaten) ausgewählt sein aus der Gruppe von Mononitrobenzol (MNB), Dinitrobenzolen (DNB), halogenierten Mono- und Dinitrobenzolen, insbesondere mono- und dinitrierten Mono- und Dichlorbenzolen, Mononitrotoluolen (MNT), Dinitrotoluolen (DNT), Trinitrotoluol und mono- und dinitrierten Xylolen sowie deren Mischungen und Kombinationen. Besonders bevorzugt ist Mononitrobenzol.

Gemäß einer bevorzugten Ausführungsform der vorliegenden Erfindung wird als nitrierbare aromatische organische Verbindung (Aromat) Benzol eingesetzt und wird als nitrierte aromatische organische Verbindung (Nitroaromat) Mononitrobenzol (MNB) erhalten.

Gemäß einer üblichen Ausführungsform erfindungsgemäßen Verfahrens kann sich der Umsetzung und/oder Nitrierreaktion eine Abtrennung der sauren wässrigen Phase (Säurephase) und/oder eine Phasentrennung des erhaltenen Nitriergemisches in Nitrierendsäure und rohe nitrierte aromatische organische Verbindungen (Roh-Nitroaromaten) anschließen, vorzugsweise gefolgt von einer Wäsche der rohen nitrierten aromatischen organischen Verbindungen (Roh-Nitroaromaten) mit einem Waschmedium, insbesondere in einem oder mehreren Waschritten, bevorzugt mit sich anschließender Abtrennung des gebrauchten Waschmediums unter Erhalt der gewaschenen und auf diese Weise von Verunreinigungen befreiten (d. h. gereinigten) nitrierten aromatischen organischen Verbindungen (Nitroaromaten).

Gemäß einer bevorzugten Ausführungsform der vorliegenden Erfindung kann die Wäsche in mindestens zwei Waschschritten durchgeführt werden, wobei mindestens ein saurer Waschschritt ("saure Wäsche") und mindestens ein neutraler Waschschritt ("neutrale Wäsche") vorgesehen sein kann.

Bevorzugterweise kann die Wäsche (i) mindestens einen ersten, im sauren Milieu durchgeführten Waschschritt ("saure Wäsche"), vorzugsweise mit Wasser oder einer Mineralsäure als Waschmedium, (ii) mindestens einen zweiten, im alkalischen (basischen) Milieu durchgeführten Waschschritt ("basische Wäsche"), vorzugsweise mit einer Base als Waschmedium, und (iii) mindestens einen dritten, im neutralen Milieu durchgeführten Waschschritt ("neutrale Wäsche"), vorzugsweise mit Wasser als Waschmedium, umfassen (und zwar in der vorgenannten Reihenfolge bzw. Abfolge von erster bis dritter Wäsche).

Gemäß einer besonderen Ausführungsform der vorliegenden Erfindung kann das verbrauchte Waschmedium rezykliert werden und/oder im Kreislauf gefahren werden und/oder wieder in die Wäsche zurückgeführt werden, insbesondere nach Aufreinigung.

Wie zuvor also ausgeführt, umfasst die Wäsche der rohen Nitroaromaten zur Entfernung der darin gelösten und suspendierten Säuren des Nitriergemisches, der Nitrophenole und anderer saurer und sonst noch mit dem Waschmittel extrahierbaren Verunreinigungen üblicherweise drei Waschschritte (siehe z. B. F. Meissner et al., Industrial and Engineering Chemistry, Vol. 46, Seiten 718 bis 724 (1954); Ullmanns Enzyklopädie der Technischen Chemie, 4. Auflage, Bd. 17, Seiten 384 bis 386; H. Hermann et al., "Industrial Nitration of Toluene to Dinitrotoluene", ACS Symposium Series 623 (1996), Seiten 234 bis 249, Editors: L. F. Albright, R. V. C. Carr, R. J. Schmitt; US 6 288 289 B1; EP 1 816 117 B1). Als Waschmedium kann üblicherweise Wasser eingesetzt werden, wobei die Wäsche üblicherweise als flüssig/flüssig-Wäsche (d. h. bei Temperaturen, bei denen der zu waschende Nitroaromat als Flüssigkeit vorliegt) durchgeführt wird. Weiterhin kann gemäß der vorliegenden Erfindung auch das Brüdenkondensat aus der Wiederaufkonzentrierung der Nitrierendsäure als Waschmedium in der sauren Wäsche (Waschschritt (i)) bzw. in der alkalischen Wäsche (Waschschritt (ii)) eingesetzt werden.

Wie zuvor ausgeführt, umfasst die dreischrittige Wäsche üblicherweise die folgenden Schritte:
(i) eine saure Wäsche mit Wasser zur Entfernung der gelösten und suspendierten Mineralsäuren, wie z. B. Schwefelsäure, Salpetersäure und Nitrose ("saure Wäsche");
(ii) eine basische bzw. alkalische Wäsche in Gegenwart einer Base ("Alkaliwäsche"), wie z. B. Natriumcarbonat (Soda), Natriumbicarbonat, Natriumsulfit, Natriumhydrogensulfit, Ammoniak, Natronlauge, Kalilauge etc. (siehe z. B. US 4 482 769 A, US 4 597 875 A oder US 6 288 289 B1), zur Entfernung der im rohen Nitroaromaten gelösten schwach aciden Verunreinigungen, wie der Nitrophenole, Nitrokresole, Nitrobenzoesäuren, Abbauprodukte aus der oxidativen Zersetzung der Phenole oder von aliphatischen oder cyclischen Kohlenwasserstoffen etc., wie z. B. Oxalsäure etc., oder den asymmetrischen Isomeren beim TNT ("basische Wäsche");
(iii) eine Neutralwäsche zur Entfernung der Restspuren von Alkali und der weiteren Reduzierung der noch in Spuren im Produkt verbliebenen Verunreinigungen ("neutrale Wäsche").

Ziel dieser Waschschritte ist es insbesondere, neben einem reinen Produkt möglichst wenig Abwasser pro Tonne Produkt zu erhalten, in welchem die ausgewaschenen Verunreinigungen so vorliegen, dass ihre Entsorgung kostengünstig durchgeführt werden kann.

Vorteilhafterweise ist es gemäß einer besonderen Ausführungsform der vorliegenden Erfindung vorgesehen, dass die nach der Umsetzung und/oder Nitrierreaktion resultierende Nitrierendsäure nach Abtrennung der rohen nitrierten aromatischen organischen Verbindungen (Roh-Nitroaromaten) rezykliert wird und/oder im Kreislauf gefahren wird und/oder wieder in die Nitrierreaktion zurückgeführt wird, insbesondere nach Aufkonzentrierung und/oder nach Zusetzen von frischer Salpeter- und/oder Schwefelsäure. Auf diese Weise wird die Verfahrenseffizienz noch weiterführend gesteigert.

Weiterhin kann auch die für die Umsetzung und/oder Nitrierreaktion zugesetzte und/oder zurückgeführte Menge an nitrierten aromatischen organischen Verbindungen (Nitroaromaten) in weiten Bereichen variieren.

Gemäß der vorliegenden Erfindung ist es vorgesehen, dass die für die Umsetzung und/oder Nitrierreaktion zugesetzte und/oder zurückgeführte Menge an nitrierten aromatischen organischen Verbindungen (Nitroaromaten) derart ausgewählt wird, dass die Menge an zugesetzten und/oder zurückgeführten nitrierten aromatischen organischen Verbindungen (Nitroaromaten) eine Absenkung der Grenzflächenspannung zwischen Organphase und Säurephase bewirkt und/oder die Menge an zugesetzten und/oder zurückgeführten nitrierten aromatischen organischen Verbindungen (Nitroaromaten) eine verbesserte Dispergierbarkeit, insbesondere Emulgierbarkeit, von Organphase und Säurephase bewirkt.

Gleichermaßen kann es gemäß einer wiederum besonderen Ausführungsform der vorliegenden Erfindung vorgesehen sein, dass die für die Umsetzung und/oder Nitrierreaktion zugesetzte und/oder zurückgeführte Menge an nitrierten aromatischen organischen Verbindungen (Nitroaromaten) derart ausgewählt wird, dass die gewichtsbezogene Menge an zugesetzten und/oder zurückgeführten nitrierten aromatischen organischen Verbindungen (Nitroaromaten), bezogen auf die zu nitrierenden und/oder umzusetzenden nitrierbaren aromatischen organischen Verbindungen (Aromaten), im Bereich von 0,01 bis 60 Gew.-%, insbesondere im Bereich von 0,1 bis 50 Gew.-%, vorzugsweise im Bereich von 5 bis 45 Gew.-%, besonders bevorzugt im Bereich von 10 bis 40 Gew.-%, liegt bzw. variiert.

Ebenfalls kann es gemäß einer weiteren besonderen Ausführungsform der vorliegenden Erfindung vorgesehen sein, dass die für die Umsetzung und/oder Nitrierreaktion zugesetzte und/oder zurückgeführte Menge an nitrierten aromatischen organischen Verbindungen (Nitroaromaten) derart ausgewählt wird, dass die gewichtsbezogene Menge an zugesetzten und/oder zurückgeführten nitrierten aromatischen organischen Verbindungen (Nitroaromaten), bezogen auf die Schwefelsäure des Salpetersäure/Schwefelsäure-Nitriersäuregemisches, im Bereich von 0,01 bis 10 Gew.-%, insbesondere im Bereich von 0,2 bis 5 Gew.-%, vorzugsweise im Bereich von 0,5 bis 3 Gew.-%, besonders bevorzugt im Bereich von 1 bis 2 Gew.-%, liegt

Auch im Hinblick auf die anderweitige Verfahrensführung ist das erfindungsgemäße Verfahren flexibel und nahezu beliebig an die jeweiligen Bedingungen (z. B. apparativen Bedingungen) adaptierbar bzw. anpassbar.

So kann auch die Zuführung an nitrierten aromatischen organischen Verbindungen (Nitroaromaten) in verschiedensten Prozess-Stadien und Positionen des erfindungsgemäßen Verfahrens erfolgen. Insbesondere kann es vorgesehen sein, dass die für die Umsetzung und/oder Nitrierreaktion zugesetzten und/oder zurückgeführten nitrierten aromatischen organischen Verbindungen (Nitroaromaten) mindestens einer der nachfolgenden Positionen (i) bis (iv) zugesetzt und/oder zugeführt werden: (i) der Ausgangsreaktionsmischung aller übrigen Reaktanden; und/oder (ii) der Schwefelsäure des Salpetersäure/Schwefelsäure-Nitriersäuregemisches, insbesondere vor Herstellung des Salpetersäure/Schwefelsäure-Nitriersäuregemisches; und/oder (iii) dem Salpetersäure/Schwefelsäure-Nitriersäuregemisch; und/oder (iv) den zu nitrierenden nitrierbaren aromatischen organischen Verbindungen (Aromaten). Grundsätzlich kommen auch Kombinationen von zwei oder mehreren dieser Varianten in Betracht.

Weiterhin kann auch die Entnahme an zurückzuführenden nitrierten aromatischen organischen Verbindungen (Nitroaromaten) in verschiedensten Prozess-Stadien und Positionen des erfindungsgemäßen Verfahrens erfolgen. Insbesondere kann es dabei vorgesehen sein, dass die für die Umsetzung und/oder Nitrierreaktion zugesetzten und/oder zurückgeführten nitrierten aromatischen organischen Verbindungen (Nitroaromaten) mindestens aus einer der nachfolgenden Positionen (i) bis (iv) stammen: (i) den rohen nitrierten aromatischen organischen Verbindungen (Roh-Nitroaromaten), vorzugsweise nach Abtrennung der sauren wässrigen Phase (Säurephase) und/oder nach Phasentrennung des erhaltenen Nitriergemisches in Nitrierendsäure und rohe nitrierte aromatische organische Verbindungen (Roh-Nitroaromaten); und/oder (ii) den gewaschenen nitrierten aromatischen organischen Verbindungen (Nitroaromaten), insbesondere nach der sauren oder neutralen Wäsche; und/oder (iii) den gewaschenen, insbesondere nach der sauren oder neutralen Wäsche erhaltenen, sowie gestrippten oder destillierten oder getrockneten nitrierten aromatischen organischen Verbindungen (Nitroaromaten); und/oder (iv) den nach der Aufkonzentrierung der Nitrierendsäure im Brüdenkondensat anfallenden nitrierten aromatischen organischen Verbindungen (Nitroaromaten). Grundsätzlich kommen auch hier Kombinationen von zwei oder mehreren dieser Varianten in Betracht.

Vorteilhafterweise kann es gemäß einer besonderen Ausführungsform der vorliegenden Erfindung vorgesehen sein, dass die für die Umsetzung und/oder Nitrierreaktion zugesetzten und/oder zurückgeführten nitrierten aromatischen organischen Verbindungen (Nitroaromaten) sowohl der Organphase als auch der Säurephase der Ausgangsreaktionsmischung zugeführt und/oder zugesetzt werden. Auf diese Weise wird ein besonders schnelles und effizientes Verteilungsgleichgewicht der zugesetzten und/oder zurückgeführten nitrierten aromatischen organischen Verbindungen (Nitroaromaten) in den beiden vorgenannten Phasen (Organphase einerseits und Säurephase andererseits) bewirkt. Bei dieser Ausführungsform kann es weiter insbesondere vorgesehen, dass der Organphase, bezogen auf die Organphase, 0,1 bis 35 Gew.-%, insbesondere 10 bis 25 Gew.-%, nitrierte aromatische organische Verbindungen (Nitroaromaten) zugesetzt werden und/oder dass der Säurephase, bezogen auf die Säurephase, 0,01 bis 3 Gew.-%, insbesondere 0,1 bis 2 Gew.-%, vorzugsweise 0,5 bis 1,5 Gew.-%, besonders bevorzugt 1,1 bis 1,5 Gew.-%, nitrierte aromatische organische Verbindungen (Nitroaromaten) zugesetzt werden.

Gemäß einer besonderen Ausführungsform des erfindungsgemäßen Verfahrens kann insbesondere derart vorgegangen werden, dass die nach der Umsetzung und/oder Nitrierreaktion resultierende Nitrierendsäure nach Abtrennung der rohen nitrierten aromatischen organischen Verbindungen (Roh-Nitroaromaten) und nach anschließender Aufkonzentrierung und gegebenenfalls Zusetzen von frischer Salpeter- und/oder Schwefelsäure rezykliert wird und/oder im Kreislauf gefahren wird und/oder wieder in die Nitrierreaktion zurückgeführt wird, wobei die für die Umsetzung und/oder Nitrierreaktion zugesetzten und/oder zurückgeführten nitrierten aromatischen organischen Verbindungen (Nitroaromaten) der aufkonzentrierten und gegebenenfalls mit frischer Salpeter- und/oder Schwefelsäure versetzten Nitrierendsäure und/oder den zu nitrierenden nitrierbaren aromatischen organischen Verbindungen (Aromaten) zugesetzt und/oder zugeführt werden. Insbesondere können bei dieser besonderen Ausführungsform die nitrierbaren aromatischen organischen Verbindungen (Aromaten) vorzugsweise unmittelbar vor Reaktionsbeginn und/oder als zeitlich letzte Reaktionskomponente (Reaktand) zugesetzt und/oder zugeführt werden, vorzugsweise unmittelbar vor der die Umsetzung und/oder Nitrierreaktion auslösenden Erstdispergierung.

Gemäß einer wiederum weiteren besonderen Ausführungsform des erfindungsgemäßen Verfahrens kann insbesondere derart vorgegangen werden, dass die nach der Umsetzung und/oder Nitrierreaktion resultierende Nitrierendsäure nach Abtrennung der rohen nitrierten aromatischen organischen Verbindungen (Roh-Nitroaromaten) und nach anschließender Aufkonzentrierung als Kreislaufsäure rezykliert wird und/oder im Kreislauf gefahren wird und/oder wieder in die Nitrierreaktion zurückgeführt wird, wobei zunächst eine Dispersion aus aufkonzentrierter Kreislaufsäure und zu nitrierenden nitrierbaren aromatischen organischen Verbindungen (Aromaten) sowie nitrierten aromatischen organischen Verbindungen (Nitroaromaten) erzeugt wird, wobei der Dispersion anschließend Salpetersäure zugesetzt, insbesondere zudispergiert, wird und auf diese Weise die Nitrierreaktion initiiert wird. Insbesondere kann bei dieser besonderen Ausführungsform die Salpetersäure vorzugsweise unmittelbar vor Reaktionsbeginn und/oder als zeitlich letzte Reaktionskomponente (Reaktand) zugesetzt und/oder zugeführt werden, vorzugsweise unmittelbar vor der die Umsetzung und/oder Nitrierreaktion auslösenden Erstdispergierung.

Wie zuvor ausgeführt, ist im Hinblick auf die Verfahrensführung das erfindungsgemäße Verfahren flexibel und nahezu beliebig an die jeweiligen Bedingungen (z. B. apparativen Bedingungen) adaptierbar bzw. anpassbar. In diesem Zusammenhang kann auch die (Reaktions-)Starttemperatur in weiten Bereichen variieren.

Üblicherweise kann es dabei vorgesehen sein, dass die Starttemperatur für die Umsetzung und/oder Nitrierreaktion in einem Temperaturbereich von 70 °C bis 120 °C, insbesondere 80 °C bis 120 °C, vorzugsweise 80 °C bis 110 °C, besonders bevorzugt 85 °C bis 105 °C, ausgewählt wird.

Wie zuvor im Zusammenhang mit den Vorteilen und Besonderheiten des erfindungsgemäßen Verfahrens erläutert, kann es gemäß einer vorteilhaften Ausführungsform des erfindungsgemäßen Verfahrens vorgesehen sein, dass die Starttemperatur für die Umsetzung und/oder Nitrierreaktion höchstens 120 °C, insbesondere höchstens 100 °C, vorzugsweise höchstens 95 °C, besonders bevorzugt höchstens 90 °C, beträgt.

Insbesondere führt die erfindungsgemäße Verfahrensführung dazu, dass die Nitrierreaktion auch mit im Vergleich zum Stand der Technik geringeren Starttemperaturen zu starten ist (und zwar, ohne dass längere Reaktions- oder Verweilzeiten als bei Verfahren nach dem Stand der Technik mit höheren Starttemperaturen, aber ansonsten gleichen Bedingungen benötigt werden). Insbesondere lässt sich durch die erfindungsgemäße Verfahrensführung erreichen, dass auch bei (Reaktions-)Starttemperaturen unterhalb von 100 °C, bevorzugt unterhalb von 95 °C, besonders bevorzugt unterhalb von 90 °C, die Nitrierung derart initiiert bzw. gestartet werden kann, wobei keine längeren Reaktions- bzw. Verweilzeiten als bei Verfahren nach dem Stand der Technik mit höheren Starttemperaturen, aber ansonsten gleichen Bedingungen benötigt werden.

Was das erfindungsgemäß als Nitrierreagens eingesetzte Salpetersäure/Schwefelsäure-Nitriersäuregemisch anbelangt, so kann es sich insbesondere um ein wässriges Salpetersäure/Schwefelsäure-Nitriersäuregemisch handeln.

Üblicherweise ist es dabei vorgesehen, dass das eingesetzte Salpetersäure/Schwefelsäure-Nitriersäuregemisch, bezogen auf das Salpetersäure/Schwefelsäure-Nitriersäuregemisch, Schwefelsäure in Mengen von 60 bis 79 Gew.-%, insbesondere 62 bis 75 Gew.-%, vorzugsweise 65 bis 72 Gew.-%, und Salpetersäure in Mengen von 1 bis 8 Gew.-%, insbesondere 2 bis 6 Gew.-%, vorzugsweise 3 bis 5 Gew.-%, enthält. Der auf 100 Gew.-% zu vervollständigende Anteil entfällt dabei auf Wasser.

Gemäß einer besonderen Ausführungsform des erfindungsgemäßen Verfahrens kann insbesondere derart vorgegangen werden, dass das Salpetersäure/Schwefelsäure-Nitriersäuregemisch in derartigen Mengen eingesetzt wird, dass das stöchiometrische Verhältnis von zu nitrierenden nitrierbaren aromatischen organischen Verbindungen (Aromaten) zu in dem eingesetzten Salpetersäure/Schwefelsäure-Nitriersäuregemisch vorliegender Salpetersäure im Bereich von 1,0 bis 1,5, insbesondere im Bereich von 1,05 bis 1,15, liegt.

Entsprechend einer vorteilhaften Ausführungsform des erfindungsgemäßen Verfahrens kann insbesondere derart vorgegangen werden, dass das Salpetersäure/Schwefelsäure-Nitriersäuregemisch in derartigen Mengen eingesetzt wird, dass das volumenbezogene Phasenverhältnis von Nitrierendsäure zu nitrierten organischen Verbindungen (Nitroaromaten) im Bereich von 3: 1 bis 25 : 1, insbesondere im Bereich von 4 : 1 bis 15 : 1, vorzugsweise im Bereich von 5 : 1 bis 8 : 1, liegt.

Das erfindungsgemäße Verfahren bzw. die Umsetzung und/oder Nitrierreaktion wird in einem Rohrreaktor durchgeführt.

Gemäß einer Ausführungsform des erfindungsgemäßen Verfahrens kann insbesondere derart vorgegangen werden, dass das Verfahren bzw. die Umsetzung und/oder Nitrierreaktion in einem Rohrreaktor durchgeführt wird, wobei die Reaktionsdauer und/oder die Verweilzeit der Reaktionsmischung im Rohrreaktor derart ausgewählt wird, dass die Salpetersäure des Salpetersäure/Schwefelsäure-Nitriersäuregemisches zu mindestens 98 %, insbesondere zu mindestens 99 %, vorzugsweise zu mindestens 99,5 %, umgesetzt ist.

Das Verfahren bzw. die Umsetzung und/oder Nitrierreaktion wird in einem Rohrreaktor durchgeführt. Dabei kann die Reaktionsdauer und/oder die Verweilzeit der Reaktionsmischung im Rohrreaktor 10 bis 180 Sekunden, insbesondere 30 bis 180 Sekunden, bevorzugt 40 bis 120 Sekunden, besonders bevorzugt 60 bis 90 Sekunden, betragen. Weiterhin kann bei dieser Ausführungsform derart vorgegangen werden, dass die Reaktionsdauer und/oder die Verweilzeit der Reaktionsmischung im Rohrreaktor 180 Sekunden, insbesondere 120 Sekunden, nicht übersteigt. Des Weiteren kann bei dieser Ausführungsform die Strömungsgeschwindigkeit der Reaktionsmischung im Rohrreaktor derart ausgewählt werden, dass eine Pfropfenströmung (Plug-flow-Bedingungen), insbesondere ohne Rückvermischung, vorliegt. Insbesondere kann es bei dieser Ausführungsform vorgesehen sein, dass die Reaktionsmischung den Rohrreaktor mit Pfropfenströmung (Plug-flow-Bedingungen), insbesondere ohne Rückvermischung, durchströmt. Insbesondere kann die Strömungsgeschwindigkeit der Reaktionsmischung im Rohrreaktor 0,01 bis 10 m/s, insbesondere 0,1 bis 5 m/s, bevorzugt 0,2 bis 3 m/s, besonders bevorzugt 0,5 bis 2 m/s, noch mehr bevorzugt 0,8 bis 1,5 m/s, betragen.

Gemäß einer besonderen Ausführungsform des erfindungsgemäßen Verfahrens kann insbesondere derart vorgegangen werden, dass der Rohrreaktor mit einem oder mehreren, vorzugsweise mit mehreren Mischelementen (Dispergierelementen), insbesondere zum Eintrag von zusätzlicher Mischenergie, ausgestattet ist. Bei dieser Ausführungsform können die Mischelemente insbesondere als Bleche, insbesondere Prall- oder Umlenkbleche, als Blenden, als statische Mischer oder als Stromteiler ausgebildet sein. Insbesondere kann es vorgesehen sein, dass durch die Mischelemente eine Mischenergie von 10 bis 1000 Joule/Liter, bevorzugt 10 bis 500 Joule/Liter, besonders bevorzugt 20 bis 200 Joule/Liter, eingetragen wird. Weiterhin kann es insbesondere vorgesehen sein, dass der Druckabfall pro Mischelement 0,1 bar bis 3,0 bar, bevorzugt 0,3 bis 1,5 bar, besonders bevorzugt 0,3 bis 0,8 bar, beträgt. Bevorzugt kann es bei dieser Ausführungsform vorgesehen sein, dass die Mischelemente derart im Rohrreaktor angeordnet sind, dass in den ersten 10 bis 30 Vol.-% des Reaktors der Umsatz der Salpetersäure des Salpetersäure/ Schwefelsäure-Nitriersäuregemisches mindestens 40 %, insbesondere mindestens 50 %, vorzugsweise mindestens 60 %, beträgt. Ebenfalls bevorzugt kann es bei dieser Ausführungsform vorgesehen sein, dass die Mischelemente derart im Rohrreaktor angeordnet sind, dass der Umsatz an eingesetzter Salpetersäure am Ende des Rohrreaktors mindestens 98 %, bevorzugt mindestens 99 %, besonders bevorzugt mindestens 99,5 %, beträgt. Schließlich kann es gemäß einer weiteren besonderen Ausführungsform des erfindungsgemäßen Verfahrens vorgesehen sein, dass dem Rohrreaktor eine Dispergiervorrichtung, vorzugsweise ein Mischorgan, insbesondere zur Erzeugung einer Dispersion oder Emulsion, insbesondere der Ausgangsreaktionsmischung oder des Nitriergemisches, vorgeschaltet ist. Bei dieser Ausführungsform kann die Dispergiervorrichtung, insbesondere das Mischorgan, als ein Rührkessel, ein Strahlmischer (Jet-Mixer) oder eine Pumpe, insbesondere eine Kreiselpumpe, ausgebildet sein. Gemäß einer besonderen Variante dieser Ausführungsform kann dabei die Dispergiervorrichtung, insbesondere das Mischorgan, als eine Pumpe, insbesondere eine Kreiselpumpe, ausgebildet sein. Gemäß einer anderen besonderen Variante dieser Ausführungsform kann dabei die Dispergiervorrichtung, insbesondere das Mischorgan, als ein Strahlmischer (Jet-Mixer) ausgebildet sein; insbesondere kann dabei der Strahlmischer einen vorzugsweise zentralen Treibstahl und einen den Treibstrahl umgebendes Medium, insbesondere in Form eines Ringstrahls, erzeugen. Insbesondere kann es bei dieser Ausführungsform vorgesehen sein, dass die Dispergiervorrichtung, insbesondere das Mischorgan, dem Rohrreaktor vorgeschaltet, vorzugsweise unmittelbar vorgeschaltet, ist, insbesondere wobei die Dispergiervorrichtung in den Reaktor übergeht oder aber insbesondere wobei die Dispergiervorrichtung in den Reaktor integriert ist und/oder Bestandteil des Reaktors ist.

In besonders bevorzugter Weise wird das erfindungsgemäße Verfahren wie folgt durchgeführt, wobei das Verfahren beispielhaft für die Nitrierung von Benzol zu Nitrobenzol beschrieben wird. Das erfindungsgemäße Verfahren kann aber insbesondere auch für alle anderen schwer in Schwefelsäure bzw. Nitriersäure dispergierbare Aromaten (wie z. B. Toluol, Xylole, Chlorbenzole etc.) angewendet werden.

Das einer adiabatischen Nitrierung von Benzol zu Nitrobenzol zugrundeliegende Verfahren und der dafür verwendete Reaktor werden beispielhaft in der EP 1 272 268 A2, in der EP 1 291 078 A2 und in der EP 2 168 942 A1 beschrieben.

Ganz allgemein kann bei einer adiabatischen Nitrierung, wie sie auch im erfindungsgemäßen Verfahren zum Einsatz kommt, eine Mischsäure (Nitriersäuregemisch bzw. Nitriersäure), typischerweise z. B. mit einem gewichtsbezogenen Gehalt an Schwefelsäure von 60 bis 79 Gew.-%, insbesondere von 62 bis 75 Gew.-%, vorzugsweise von 65 bis 72 Gew.-%, und mit einem gewichtsbezogenen Gehalt an Salpetersäure von 1 bis 8 Gew.-%, bevorzugt 2 bis 6 Gew.-%, besonders bevorzugt von 3 bis 5 Gew.-%, mit dem zu nitrierenden Benzol, bevorzugt mit einem stöchiometrischen Verhältnis von Benzol zu Salpetersäure von 1,0 bis 1,5, bevorzugt von 1,05 bis 1,15, in einer Dispergiervorrichtung vermischt werden. Das Phasenverhältnis zwischen Organphase und Säurephase im Nitriergemisch wird durch die Konzentration an Salpetersäure in der eingesetzten Mischsäure festgelegt: Mit einer Mischsäure mit 4,5 Gew.% an Salpetersäure ergibt sich bei einem Überschuss an Benzol von 10 % auf Gewichtsbasis ein Verhältnis von Mischsäure/Benzol von 16,3. Am Ende der Umsetzung wird dann ein Nitriergemisch, in welchem die gesamte Reaktionswärme gespeichert wird, mit einem Verhältnis Endsäure/Produkt von ca. 10,3 erhalten. Der Temperaturanstieg im Nitriergemisch, d.h. die Differenz (ΔT) zwischen End- und Starttemperatur, ist bei einer Umsetzung der Salpetersäure zu Produkt von mehr als 99 %, eindeutig durch das Phasenverhältnis festgelegt. Wird das Phasenverhältnis kleiner, z.B. durch einen höheren Gehalt an Salpetersäure in der Mischsäure, wird die Differenz (ΔT) zwischen End- und Starttemperatur größer und umgekehrt.

Nach dem Vermischen der Reaktionspartner wird das zweiphasige Gemisch aus Organ- und Säurephase derart dispergiert, dass die Nitrierung in einem Rohrreaktor startet, was erkennbar ist an einem steilen Temperaturanstieg im Nitriergemisch. Die Starttemperatur ergibt sich als Mischtemperatur der einzelnen Eduktströme und bewegt sich für eine adiabatische Nitrierung, insbesondere von Benzol zu Nitrobenzol, typischerweise im Bereich von 70 bis 120 °C, insbesondere im Bereich von 80 bis 120 °C, vorzugsweise im Bereich von 80 bis 110 °C, besonders bevorzugt im Bereich von 85 bis 105 °C. Insbesondere beträgt die Starttemperatur für die Umsetzung und/oder Nitrierreaktion höchstens 120 °C, insbesondere höchstens 100 °C, vorzugsweise höchstens 95 °C, besonders bevorzugt höchstens 90 °C.

Üblicherweise kann die Verweilzeit des Nitriergemisches im Rohrreaktor, in welcher am Ende mindestens 98 %, bevorzugt mehr als 99 %, der eingesetzten Salpetersäure umgesetzt sind, 30 bis 180 Sekunden, bevorzugt nicht mehr als 120 Sekunden, besonders bevorzugt 60 bis 90 Sekunden, betragen. Die Fließgeschwindigkeit des Nitriergemisches im Rohr kann dabei, 0,1 bis 5,0 m/s, bevorzugt 0,2 bis 3,0 m/s, besonders bevorzugt 0,5 bis 2,0 m/s, ganz besonders bevorzugt 0,8 bis 1,5 m/s, betragen, damit eine sogenannte Pfropfenströmung (Plug-flow-Bedingungen) ohne Rückvermischung im Rohrreaktor vorliegt. Durch Festlegung einer Stundenleistung an Produkt, Angaben eines Gehalts an Salpetersäure in der Mischsäure, Verweilzeit und Fließgeschwindigkeit im Rohrreaktor ist die Dimensionierung des Rohrreaktors eindeutig festgelegt.

Gemäß einer besonderen Ausführungsform kann zu Beginn bevorzugt die Mischsäure durch Zusammenführung von Schwefelsäure und Salpetersäure, hergestellt werden. In einem zweiten Schritt kann dann in diese Mischsäure der zu nitrierende Aromat dispergiert werden. Die Starttemperatur resultiert dann als die Mischtemperatur aus den einzelnen Eduktströmen. Die Temperaturen der Eduktströme werden bevorzugt gezielt derart eingestellt, dass nach dem Zusammenmischen die gewünschte Starttemperatur vorliegt.

Neben der Auswahl einer geeigneten Starttemperatur, Festlegung der Temperaturdifferenz (ΔT) zwischen End- und Starttemperatur sowie der Endtemperatur des Nitriergemisches selbst ist eine weitere Voraussetzung für einen weitgehend vollständigen Umsatz der Salpetersäure unter Einhaltung der vorgegebenen Verweilzeiten eine derartige Dispergierung des zu nitrierenden Aromaten in der Mischsäure, dass unmittelbar nach der gezielt vorgenommenen ersten Dispergierung die Nitrierung so einsetzt, dass der gewünschte Temperaturanstieg im Nitriergemisch erfolgt (was dem Fachmann aus dem Stand der Technik bekannt und geläufig ist). Zu diesem Zwecke kann der zu nitrierende Aromat z. B. durch entsprechend geformte Düsen in der Mischsäure dispergiert werden (vgl. z. B. EP 0 373 966 A2, EP 0 436 443 A2 oder EP 0 708 076 A2) oder das Nitriergemisch durch statische Mischer (vgl. z. B. EP 0 489 211 A1 oder EP 0 779 270 A1) oder durch Strahlmischer (vgl. z. B. EP 0 771 783 A1) oder durch Einsatz speziell geformter Blenden (vgl. z. B. EP 1 272 268 A2 oder EP 1 291 078 A2), welche bevorzugt auch bei der Redispergierung zum Einsatz kommen, erstmals dispergiert werden.

Unabhängig von der verwendeten Dispergiertechnik liegen bei der erstmaligen Dispergierung der Phasen ineinander, z. B. von Benzol in die in großem Überschuss vorliegende Mischsäure, die ungünstigsten Grenzflächenspannungen zwischen den Phasen vor, wodurch eine optimale Dispergierung behindert wird. Zusätzlich neigen die durch Energieeintrag erzeugten Dispersionen der reinen zu nitrierenden Aromaten in Mischsäure zu schneller Koaleszenz.

Die Grenzflächenspannungen zwischen Nitroaromat und wässriger Phase (z. B. Nitrobenzol und Wasser) sind deutlich geringer als die Grenzflächenspannungen zwischen dem zu nitrierenden Aromat (z. B. Benzol) und Wasser. Durch den erfindungsgemäßen Zusatz des nitrierten Produktes vor der ersten, die Umsetzung auslösenden Erstdispergierung werden die Grenzflächenspannungen an den Phasengrenzflächen effizient und signifikant reduziert, wodurch eine verbesserte Dispergierung zum Start erreicht und die Neigung zur Koaleszenz der erstmalig erzeugten Dispersion reduziert wird.

Für die zusätzliche, erfindungsgemäß vorgesehene Zugabe des nitrierten Produktes zum Nitriergemisch vor Start der Nitrierung als weiterer Komponente neben Schwefelsäure, Salpetersäure und zu nitrierendem Aromaten (z. B. Benzol), für eine adiabatische Nitrierung in einem Rohrreaktor, insbesondere kurz vor dem Reaktionsstart, d. h. vor der ersten, die Nitrierung auslösenden Erstdispergierung, sind verschiedene Varianten in der Reihenfolge der Zugabe möglich. Das zurückzuführende Produkt kann insbesondere
a) vor Zugabe des zu nitrierenden Aromaten (z. B. Benzol) und vor Zumischen der Salpetersäure in die aufkonzentrierte und rezyklierte Schwefelsäure (Kreislaufsäure) und/oder
b) vor Zugabe des zu nitrierenden Aromaten (z. B. Benzol) kurz nach Zumischen der Salpetersäure in die Mischsäure und/oder
c) zusammen mit dem zu nitrierenden Aromaten und/oder
d) als Teilstrom der Nitrieremulsion vor der Phasentrennung in die Kreislaufsäure oder Mischsäure und/oder
e) auch als eine Kombination nach a) bis c), insbesondere derart, dass ein Teil des zugesetzten nitrierten Produktes (z. B. Nitrobenzol) in die wässrigen Phase nach a) und/oder b) und ein anderer Teil zusammen mit dem zu nitrierenden Aromat nach c) erfolgt,
erfolgen.

Die Zugabe des zu nitrierenden Aromaten als zweiter reaktiver Komponente im Nitriergemisch neben der Salpetersäure erfolgt vorteilhafterweise in den Fällen a) bis e) insbesondere stets am Schluss, vorzugsweise kurz vor der die Umsetzung auslösenden Erstdispergierung.

Neben diesen Varianten, insbesondere auch dem Zumischen des zu nitrierenden Aromaten zum Nitriergemisch als letzter Komponente vor der Erstdispergierung, ist es besonders vorteilhaft, die Salpetersäure als zweiten an der Nitrierung beteiligten Partner als letzte Komponente vor der Erstdispergierung zum Nitriergemisch zuzusetzen.

Es hat sich als vorteilhaft erwiesen, dass nach jedem Zudosieren eines Eduktes zu der aufkonzentrierten Kreislaufschwefelsäure, dem Hauptanteil im Nitriergemisch, geeignete Mischorgane (z. B. statische Mischer, Blenden Strahlmischer etc.) eine homogene Vermischung mit der Kreislaufsäure (im Fall von Salpetersäure) oder eine Vordispergierung in der Kreislaufschwefelsäure (im Fall von zurückgeführtem nitriertem Produkt oder zu nitrierendem Aromat) erfolgt derart, dass an diesen Mischorganen ein nur geringer Druckverlust entsteht,

Das in die aufkonzentrierte Kreislaufsäure, Mischsäure oder den zu nitrierenden Aromaten (z. B. Benzol) zugesetzte Produkt (z. B. Nitrobenzol) kann gleichermaßen aus verschiedenen Stufen des Prozesses entnommen werden, d. h.
A) als Rohnitrobenzol, insbesondere nach der Phasentrennung des Nitriergemisches aus Nitrierendsäure und Nitrobenzol, vor oder nach Kühlung, und vorzugsweise vor der Behandlung mit einem Waschmedium (Dieses Rohnitrobenzol enthält neben dem Produkt noch 2 bis 10 % an Benzol, Nitrophenolen und Spuren an Schwefelsäure gelöst, ca. 0,2 bis 0,25 %, und Endsäure als Mikroemulsion, aber kein Wasser); und/oder
B) als teilweise gereinigtes Nitrobenzol, frei von Säuren, nach der sauren Wäsche (Dieses Nitrobenzol aus der sauren Wäsche enthält neben den gesamten Nitrophenolen noch alles überschüssige Benzol und ist mit Wasser gesättigt.); und/oder
C) als gereinigtes Nitrobenzol nach der Neutralwäsche (Dieses Nitrobenzol aus der Neutralwäsche enthält nur noch Spuren an Nitrophenolen, ca. 2 bis 60 ppm, und noch alles überschüssige Benzol und ist ebenfalls mit Wasser gesättigt.); und/oder
D) als das bei der Aufkonzentrierung der Endsäure zur Kreislaufsäure im Brüdenkondensat anfallende Nitrobenzol (Dieses Nitrobenzol, ca. 10-15% des Produktes, aus dem Kondensat der Aufkonzentrierung der Endsäure ist quasi frei von Nitrophenolen, enthält ebenfalls noch kleine Reste an Benzol und ist ebenfalls mit Wasser gesättigt.) und/oder
E) als Endprodukt, nach Abtrennung des Überschusses an Benzol, wasserfrei oder mit Wasser gesättigt; und/oder
F) weiterhin als eine Teilentnahme von Nitrieremulsion vor der Phasentrennung mit nachfolgender Rückführung in den Nitrierprozess; und/oder
G) Kombinationen von A) bis F).

Vorteilhaft ist die Rückführung eines noch Benzol enthaltenden Nitrobenzols nach den vorgenannten Positionen A) bis C) und besonders vorteilhaft eines noch Benzol enthaltenden Nitrobenzols nach den vorgenannten Positionen B) und C), um die Bildung von Nebenprodukten, vor allem Dinitrobenzol aus Nitrobenzol, mit Spuren an Salpetersäure in der Kreislaufsäure zu minimieren.

Bei Zugabe des Produktes zu einer aufkonzentrierten Kreislaufsäure vor Zugabe der Salpetersäure bzw. zur Mischsäure werden insbesondere 0,1 bis 5,0 Gew.-%, bevorzugt 0,5 bis 3,0 Gew.-%, besonders bevorzugt 1,1 bis 2,0 Gew.-%, der Kreislaufsäure zugesetzt.

Durch Versetzen des zu nitrierenden Benzols mit Nitrobenzol derart, dass ein Gemisch von Benzol/Nitrobenzol von 0,1 bis 50 %, bevorzugt 5,0 bis 45 %, besonders bevorzugt 11 bis 40 %, an Nitrobenzol zudosiert wird, wird ebenfalls eine effiziente Absenkung der Grenzflächenspannung zwischen der Organ- und Säurephase erreicht.

Sowohl bei Zusatz des nitrierten Produktes (z. B. Nitrobenzol) direkt zur Kreislaufsäure als auch im Fall des Zusatzes des Produktes (z.B. Nitrobenzol) zu dem zu nitrierenden Aromaten ist es vorteilhaft, eine Vordispergierung des nitrierten Produktes bzw. des Gemisches aus nitriertem Produkt/zu nitrierendem Aromaten und eine gewisse Verweilzeit, deren Dauer von der Qualität der Vordispergierung der zugesetzten Organphasen in der Kreislaufsäure abhängt, vorzusehen, damit sich ein Verteilungsgleichgewicht an nitriertem Produkt zwischen Säure und Organphase einstellen kann, bevor die optimal veränderte Grenzflächenspannung zwischen den nitriertes Produkt enthaltenden beiden Phasen wirksam wird.

Zur Verkürzung dieser Verweilzeit vor der Erstdispergierung ist es besonders vorteilhaft, nitriertes Produkt (z. B. Nitrobenzol) in einer solchen Menge zur Kreislaufsäure bzw. Mischsäure zuzusetzen, dass die Löslichkeitsgrenze für das zugesetzte Produkt in der entsprechenden Säure überschritten wird. Es bildet sich dann bereits ein aus zwei Phasen bestehendes Gemisch aus nitriertem Produkt/Säure.

Bei Zugabe des zu nitrierenden Aromaten zu diesem Gemisch wird der nicht in der Säure gelöste Anteil des zugesetzten nitrierten Produktes sofort mit dem zu nitrierenden Aromat vermischt, so dass bereits vor der ersten Dispergierung ein Gemisch aus Benzol/Nitrobenzol als Organphase resultiert. Beide Phasen, welche jeweils Nitrobenzol enthalten, tragen auf diese Weise zu dem für die Erstdispergierung erwünschten reduzierten Grenzflächenspannung bei.

Eine weitere Möglichkeit zur Abkürzung der Verweilzeit im Rohrreaktor vor der Erstdispergierung und damit zur beschleunigten Erreichung eines annähernden Verteilungsgleichgewicht für das zugesetzte Produkt zwischen den beiden Phasen besteht darin, das Produkt (z. B. Nitrobenzol) sowohl über die Säurephase als auch über die Organphase dem Nitrierkreislauf zuzuführen, und zwar insbesondere derart, dass der Säurephase z. B. 0,1 bis 2,0 Gew.-%, bevorzugt 0,5 bis 1,5 Gew%, besonders bevorzugt 1,1 bis 1,5 Gew.-%, bezogen auf die Menge an Kreislaufsäure, zugesetzt werden kann und/oder dass der Organphase aus zu nitrierendem Benzol und Nitrobenzol z. B. 0,1 bis 32 Gew.-%, bevorzugt 11,0 bis 25 Gew%, an Nitrobenzol zugesetzt werden kann.

Neben der Variante der Zugabe des zu nitrierenden Aromaten als letzte Komponente vor der Erstdispergierung des vollständigen Nitriergemisches, wie vorstehend beschrieben, kann es gleichermaßen vorteilhaft sein, erst das Gemisch aus Kreislaufsäure, nitriertem Produkt und zu nitrierendem Aromat vorzudispergieren und nachfolgend als letzte Komponente die Salpetersäure zu dem Nitriergemisch zuzufügen, und zwar insbesondere derart, dass nicht nur durch geeignete Mischorgane in Bruchteilen von Sekunden die Salpetersäure homogen in der wässrigen Phase gleichmäßig verteilt wird und das vordispergierte Nitriergemisch nicht nur erhalten bleibt, sondern eine zusätzliche weitere Dispergierung und, damit verbunden, eine noch größere Austauschfläche zwischen dispergierter Organphase und homogener Säurephase erzeugt wird.

Die vorstehende Erfindung bzw. das erfindungsgemäße Verfahren ist grundsätzlich für beliebige aus dem Stand der Technik bekannte adiabatische Nitrierverfahren in Verbindung mit einem Rohrreaktor einsetzbar, bei welchen die Nitrierung mit reinen Edukten, insbesondere dem schwer zu dispergierenden Aromaten (bevorzugt Benzol) und einer reinen Mischsäure (wässriges Salpetersäure/Schwefelsäure-Nitriersäuregemisch), durch Eintrag von mechanischer Mischenergie gestartet wird, wie z. B. in der EP 0 373 966 A2, in der EP 0 436 443 A2, in der EP 0 489 211 A1, in der EP 0 708 076 A2, in der EP 0 771 783 A1, in der EP 0 779 270 A1, in der EP 1 272 268 A2, in der EP 1 291 078 A2, in der EP 2 168 942 A1, in der EP 2 354 117 A1 und in der EP 2 473 477 A1 beschrieben.

Weiterer Gegenstand der vorliegenden Erfindung - gemäß einem **zweiten** Aspekt der vorliegenden Erfindung - ist eine Produktionsanlage (Nitrieranlage bzw. Anlage) zur adiabatischen Nitrierung von nitrierbaren aromatischen organischen Verbindungen (Aromaten) zu nitrierten Produkten in Form der entsprechenden nitrierten aromatischen organischen Verbindungen (Nitroaromaten), insbesondere Produktionsanlage zur Durchführung eines Verfahrens gemäß einem der vorangehenden Ansprüche,
wobei die Produktionsanlage die folgenden Einheiten und Vorrichtungen umfasst:
(a) eine Nitriereinheit N zur Nitrierung, insbesondere adiabatischen Nitrierung, von nitrierbaren aromatischen organischen Verbindungen (Aromaten) zu nitrierten Produkten in Form der entsprechenden nitrierten aromatischen organischen Verbindungen (Nitroaromaten), insbesondere mit einem oder mehreren Reaktoren zur Durchführung der Nitrierreaktion, wobei die Nitriereinheit N als Reaktor mindestens einen Rohrreaktor umfasst;
(b) in Produktionslinie stromabwärts zur Nitriereinheit N angeordnet, mindestens eine Abtrennvorrichtung S, insbesondere eine Scheidevorrichtung (Scheider), zur Abtrennung der Nitrierendsäure von den nitrierten Rohprodukten;
(c) in Produktionslinie stromabwärts zur Nitriereinheit N und zur Abtrennvorrichtung S angeordnet, mindestens eine Wascheinheit W zur Durchführung einer Wäsche der nitrierten Rohprodukte mit einem Waschmedium, insbesondere in einem oder mehreren Waschschritten;
(d) in Produktionslinie stromabwärts zur Wascheinheit W angeordnet, eine Abtrennvorrichtung, insbesondere eine Scheidevorrichtung (Scheider), zur Abtrennung der gewaschenen nitrierten Produkte vom Waschmedium;
wobei die Produktionsanlage außerdem mindestens eine Rückführvorrichtung R zur teilweisen Rückführung der nitrierten Produkte in die Nitriereinheit N, insbesondere in die Ausgangsreaktionsmischung der Nitriereinheit N, umfasst.

Gemäß der vorliegenden Erfindung ist es vorgesehen, dass die Nitriereinheit als Reaktor mindestens einen Rohrreaktor umfasst.

Insbesondere kann es gemäß einer bevorzugten Ausführungsform der erfindungsgemäßen Produktionsanlage vorgesehen sein, dass der Rohrreaktor mit einem oder mehreren, vorzugsweise mit mehreren Mischelementen (Dispergierelementen), insbesondere zum Eintrag von zusätzlicher Mischenergie, ausgestattet ist. Bei dieser Ausführungsform können die Mischelemente insbesondere als Bleche, insbesondere Prall- oder Umlenkbleche, als Blenden, als statische Mischer oder als Stromteiler ausgebildet sein. Weiterhin können bei dieser Ausführungsform die Mischelemente insbesondere derart ausgebildet sein, dass durch die Mischelemente im Betriebszustand eine Mischenergie von 10 bis 1000 Joule/Liter, bevorzugt 10 bis 500 Joule/Liter, besonders bevorzugt 20 bis 200 Joule/Liter, eingetragen wird. Weiterhin kann es bei dieser Ausführungsform vorgesehen sein, dass im Betriebszustand der Druckabfall pro Mischelement 0,1 bar bis 3,0 bar, bevorzugt 0,3 bis 1,5 bar, besonders bevorzugt 0,3 bis 0,8 bar, beträgt. Weiterhin kann es gemäß einer besonderen Variante dieser Ausführungsform vorgesehen sein, dass die Mischelemente derart im Rohrreaktor angeordnet sind, dass im Betriebszustand in den ersten 10 bis 30 Vol.-% des Reaktors der Umsatz der Salpetersäure des Salpetersäure/Schwefelsäure-Nitriersäuregemisches mindestens 40 %, insbesondere mindestens 50 %, vorzugsweise mindestens 60 %, beträgt. Schließlich kann es gemäß einer besonderen Variante dieser Ausführungsform vorgesehen sein, dass die Mischelemente derart im Rohrreaktor angeordnet sind, dass der Umsatz an eingesetzter Salpetersäure am Ende des Rohrreaktors mindestens 98 %, bevorzugt mindestens 99 %, besonders bevorzugt mindestens 99,5 %, beträgt.

Entsprechend einer besonderen Ausgestaltung der erfindungsgemäßen Produktionsanlage kann es weiterhin vorgesehen sein, dass dem oder den Rohrreaktoren der Nitriereinheit eine Dispergiervorrichtung, vorzugsweise ein Mischorgan, insbesondere zur Erzeugung einer Dispersion oder Emulsion, insbesondere der Ausgangsreaktionsmischung oder des Nitriergemisches, vorgeschaltet ist.

Bei dieser Ausführungsform kann die Dispergiervorrichtung, insbesondere das Mischorgan, als ein Rührkessel, ein Strahlmischer (Jet-Mixer) oder eine Pumpe, insbesondere eine Kreiselpumpe, ausgebildet sein. Gemäß einer besonderen Variante dieser Ausführungsform kann dabei die Dispergiervorrichtung, insbesondere das Mischorgan, als eine Pumpe, insbesondere eine Kreiselpumpe, ausgebildet sein. Gemäß einer anderen besonderen Variante dieser Ausführungsform kann dabei die Dispergiervorrichtung, insbesondere das Mischorgan, als ein Strahlmischer (Jet-Mixer) ausgebildet ist, insbesondere wobei der Strahlmischer einen vorzugsweise zentralen Treibstahl und einen den Treibstrahl umgebendes Medium, insbesondere in Form eines Ringstrahls, erzeugt. Insbesondere kann es bei dieser Ausführungsform vorgesehen sein, dass die Dispergiervorrichtung, insbesondere das Mischorgan, dem Rohrreaktor vorgeschaltet, vorzugsweise unmittelbar vorgeschaltet, ist, insbesondere wobei die Dispergiervorrichtung in den Reaktor übergeht oder aber insbesondere wobei die Dispergiervorrichtung in den Reaktor integriert ist und/oder Bestandteil des Reaktors ist.

Was die in Produktionslinie stromabwärts zur Nitriereinheit und zur gegebenenfalls vorhandenen Abtrennvorrichtung angeordnete Wascheinheit anbelangt, so kann diese Wascheinheit typischerweise
- mindestens eine Dispergiervorrichtung, insbesondere mindestens ein Mischorgan, zum Inkontaktbringen und Emulgieren der nitrierten Rohprodukte einerseits und einem Waschmedium andererseits und,
- stromabwärts zur Dispergiervorrichtung angeordnet, einen Rohrreaktor zur Einspeisung der in der Dispergiervorrichtung erzeugten Emulsion von nitrierten Rohprodukten einerseits und Waschmedium andererseits; insbesondere wobei der Rohrreaktor derart ausgebildet ist, dass während des Durchtritts der Emulsion durch den Rohrreaktor eine Entfernung der in den nitrierten Rohprodukten anfänglich vorhandenen Verunreinigungen ermöglicht wird und/oder dass während des Durchtritts der Emulsion durch den Rohrreaktor die in den nitrierten Rohprodukten anfänglich vorhandenen Verunreinigungen in das Waschmedium überführt und/oder hierdurch neutralisiert werden,
umfassen.

Es versteht sich von selbst, dass zusätzlich auch noch herkömmliche Waschvorrichtungen (z. B. Mixer/Settler-Vorrichtungen, Extraktionskolonnen etc.) vorhanden sein können.

Üblicherweise kann die Wascheinheit zur Durchführung Wäsche in mindestens zwei Waschschritten ausgebildet sein, insbesondere mindestens einer sauren Wäsche und mindestens eine neutralen Wäsche.

Gemäß ein bevorzugten Ausführungsform kann es insbesondere vorgesehen sein, dass die Wascheinheit (W) zur Durchführung einer Wäsche mit mindestens drei Waschschritten ausgebildet ist. Insbesondere kann dabei die mindestens drei Waschschritte umfassende Wäsche umfassen: (i) mindestens einen ersten, im sauren Milieu durchgeführten Waschschritt ("saure Wäsche"), vorzugsweise mit Wasser oder einer Mineralsäure als Waschmedium, (ii) mindestens einen zweiten, im alkalischen (basischen) Milieu durchgeführten Waschschritt ("basische Wäsche"), vorzugsweise mit einer Base als Waschmedium, und (iii) mindestens einen dritten, im neutralen Milieu durchgeführten Waschschritt ("neutrale Wäsche"), vorzugsweise mit Wasser als Waschmedium.

Wie zuvor im Zusammenhang mit dem erfindungsgemäßen Verfahren erläutert, ist im Hinblick auf die Verfahrensführung das erfindungsgemäße Verfahren flexibel und nahezu beliebig an die jeweiligen Bedingungen (z. B. apparativen Bedingungen) adaptierbar. So können -wie zuvor ausgeführt - insbesondere Zuführung und Entnahme an nitrierten aromatischen organischen Verbindungen (Nitroaromaten) in verschiedensten Prozess-Stadien und Positionen des erfindungsgemäßen Verfahrens erfolgen. Dies findet auch im Hinblick auf die erfindungsgemäße Produktionsanlage seinen Niederschlag.

So kann gemäß einer besonderen Ausführungsform der vorliegenden Erfindung die in der erfindungsgemäßen Produktionsanlage vorgesehene Rückführvorrichtung zur teilweisen Rückführung der nitrierten Produkte in die Nitriereinheit insbesondere derart ausgebildet und/oder angeordnet sein, dass die teilweise rückzuführenden nitrierten Produkte mindestens aus einer der nachfolgenden Positionen (i) bis (iv) des Produktionsstroms entnommen werden: (i) den rohen nitrierten aromatischen organischen Verbindungen (Roh-Nitroaromaten), vorzugsweise nach Abtrennung der sauren wässrigen Phase (Säurephase) und/oder nach Phasentrennung des erhaltenen Nitriergemisches in Nitrierendsäure und rohe nitrierte aromatische organische Verbindungen (Roh-Nitroaromaten); und/oder (ii) den gewaschenen nitrierten aromatischen organischen Verbindungen (Nitroaromaten), insbesondere nach der sauren oder neutralen Wäsche; und/oder (iii) den gewaschenen, insbesondere nach der sauren oder neutralen Wäsche erhaltenen, sowie gestrippten oder destillierten oder getrockneten nitrierten aromatischen organischen Verbindungen (Nitroaromaten); und/oder (iv) den nach der Aufkonzentrierung der Nitrierendsäure im Brüdenkondensat anfallenden nitrierten aromatischen organischen Verbindungen (Nitroaromaten). Grundsätzlich kommen auch Kombinationen von zwei oder mehreren dieser Varianten in Betracht.

Des Weiteren kann gemäß einer weiteren besonderen Ausführungsform der vorliegenden Erfindung die in der erfindungsgemäßen Produktionsanlage vorgesehene Rückführvorrichtung zur teilweisen Rückführung der nitrierten Produkte in die Nitriereinheit insbesondere derart ausgebildet und/oder angeordnet sein, dass die teilweise rückzuführenden nitrierten Produkte mindestens einer der nachfolgenden Positionen (i) bis (iv) des Produktionsstroms zugesetzt und/oder zugeführt werden: (i) der Ausgangsreaktionsmischung aller übrigen Reaktanden; und/oder (ii) der Schwefelsäure des Salpetersäure/Schwefelsäure-Nitriersäuregemisches, insbesondere vor Herstellung des Salpetersäure/Schwefelsäure-Nitriersäuregemisches; und/oder (iii) dem Salpetersäure/Schwefelsäure-Nitriersäuregemisch; und/oder (iv) den zu nitrierenden nitrierbaren aromatischen organischen Verbindungen (Aromaten). Grundsätzlich kommen auch hier Kombinationen von zwei oder mehreren dieser Varianten in Betracht.

Schließlich kann es gemäß einer weiteren besonderen Ausführungsform der erfindungsgemäßen Produktionsanlage vorgesehen sein, dass die Produktionsanlage außerdem mindestens eine Rezykliervorrichtung zur Rezyklierung der Nitrierendsäure umfasst. Auf diese Weise kann die Prozessökonomie und Prozesseffizienz noch weiterführend gesteigert werden. Insbesondere kann bei dieser besonderen Ausführungsform die Rezykliervorrichtung eine Einrichtung zur Aufkonzentrierung der Nitrierendsäure und gegebenenfalls eine Einrichtung zum Zusetzen von frischer Salpeter- und/oder Schwefelsäure umfassen.

Bezüglich weitergehender Einzelheiten zu der erfindungsgemäßen Produktionsanlage kann zur Vermeidung unnötiger Wiederholungen auf die obigen Ausführungen zu dem erfindungsgemäßen Verfahren verwiesen werden, welche in Bezug auf die erfindungsgemäße Produktionsanlage entsprechend gelten.

Weiterer Gegenstand der vorliegenden Erfindung - gemäß einem **dritten** Aspekt der vorliegenden Erfindung - ist die Verwendung von nitrierten aromatischen organischen Verbindungen (Nitroaromaten) als Dispergator (Dispergiermittel), insbesondere Emulgator, für Nitrierungen, insbesondere für Nitrierreaktionen der entsprechenden unnitrierten aromatischen organischen Verbindungen.

Wiederum weiterer Gegenstand der vorliegenden Erfindung gemäß diesem Erfindungsaspekt ist die Verwendung von nitrierten aromatischen organischen Verbindungen (Nitroaromaten) zur Erniedrigung der Grenzflächenspannung von Organphase und Säurephase und/oder zur Verbesserung der Dispergierbarkeit von Organphase und Säurephase bei Nitrierungen, insbesondere bei Nitrierreaktionen der entsprechenden unnitrierten aromatischen organischen Verbindungen,
wobei nitrierbare aromatische organische Verbindungen (Aromaten) in einer adiabatischen Nitrierreaktion mit einem Salpetersäure/Schwefelsäure-Nitriersäuregemisch zu den entsprechenden nitrierten aromatischen organischen Verbindungen (Nitroaromaten) umgesetzt werden;
wobei der Ausgangsreaktionsmischung, welche die nitrierbaren aromatischen organischen Verbindungen (Aromaten) und das Salpetersäure/Schwefelsäure-Nitriersäuregemisch umfasst, entsprechende nitrierte aromatische organische Verbindungen (Nitroaromaten) zugesetzt werden und die Umsetzung und/oder Nitrierreaktion in Gegenwart der nitrierten aromatischen organischen Verbindungen (Nitroaromaten) gestartet und/oder durchgeführt wird; und
wobei die erhaltenen nitrierten aromatischen organischen Verbindungen (Nitroaromaten) teilweise in die Nitrierreaktion zurückgeführt werden und die nachfolgende Umsetzung und/oder Nitrierreaktion in Gegenwart der nitrierten aromatischen organischen Verbindungen (Nitroaromaten) gestartet und/oder durchgeführt wird;
wobei die Umsetzung und/oder Nitrierreaktion unter adiabatischen Reaktionsbedingungen durchgeführt wird,
wobei die Umsetzung und/oder Nitrierreaktion in einem Rohrreaktor durchgeführt wird und
wobei die für die Umsetzung und/oder Nitrierreaktion zugesetzte und/oder zurückgeführte Menge an nitrierten aromatischen organischen Verbindungen (Nitroaromaten) derart ausgewählt wird, dass die Menge an zugesetzten und/oder zurückgeführten nitrierten aromatischen organischen Verbindungen (Nitroaromaten) eine Absenkung der Grenzflächenspannung zwischen Organphase und Säurephase bewirkt und eine verbesserte Dispergierbarkeit von Organphase und Säurephase bewirkt.

Schließlich ist wiederum weiterer Gegenstand der vorliegenden Erfindung gemäß diesem Erfindungsaspekt die Verwendung von nitrierten aromatischen organischen Verbindungen (Nitroaromaten) zur Erhöhung der Ausbeuten und/oder zur Reduktion der Nebenproduktbildung und/oder zur Verkürzung des Reaktionsgesamtdauern und/oder zur Erniedrigung der Reaktionsstarttemperaturen bei Nitrierungen, insbesondere bei Nitrierreaktionen der entsprechenden unnitrierten aromatischen organischen Verbindungen,
wobei nitrierbare aromatische organische Verbindungen (Aromaten) in einer adiabatischen Nitrierreaktion mit einem Salpetersäure/Schwefelsäure-Nitriersäuregemisch zu den entsprechenden nitrierten aromatischen organischen Verbindungen (Nitroaromaten) umgesetzt werden;
wobei der Ausgangsreaktionsmischung, welche die nitrierbaren aromatischen organischen Verbindungen (Aromaten) und das Salpetersäure/Schwefelsäure-Nitriersäuregemisch umfasst, entsprechende nitrierte aromatische organische Verbindungen (Nitroaromaten) zugesetzt werden und die Umsetzung und/oder Nitrierreaktion in Gegenwart der nitrierten aromatischen organischen Verbindungen (Nitroaromaten) gestartet und/oder durchgeführt wird; und
wobei die erhaltenen nitrierten aromatischen organischen Verbindungen (Nitroaromaten) teilweise in die Nitrierreaktion zurückgeführt werden und die nachfolgende Umsetzung und/oder Nitrierreaktion in Gegenwart der nitrierten aromatischen organischen Verbindungen (Nitroaromaten) gestartet und/oder durchgeführt wird;
wobei die Umsetzung und/oder Nitrierreaktion unter adiabatischen Reaktionsbedingungen durchgeführt wird,
wobei die Umsetzung und/oder Nitrierreaktion in einem Rohrreaktor durchgeführt wird und
wobei die für die Umsetzung und/oder Nitrierreaktion zugesetzte und/oder zurückgeführte Menge an nitrierten aromatischen organischen Verbindungen (Nitroaromaten) derart ausgewählt wird, dass die Menge an zugesetzten und/oder zurückgeführten nitrierten aromatischen organischen Verbindungen (Nitroaromaten) eine Absenkung der Grenzflächenspannung zwischen Organphase und Säurephase bewirkt und eine verbesserte Dispergierbarkeit von Organphase und Säurephase bewirkt.

Bezüglich weitergehender Einzelheiten zu den erfindungsgemäßen Verwendungen kann zur Vermeidung unnötiger Wiederholungen auf die obigen Ausführungen zu dem erfindungsgemäßen Verfahren und zu der erfindungsgemäßen Produktionsanlage verwiesen werden, welche in Bezug auf die erfindungsgemäßen Verwendungen entsprechend gelten.

Weitere Vorteile, Eigenschaften Aspekte und Merkmale der vorliegenden Erfindung ergeben sich aus der nachfolgenden Beschreibung der in den Figurendarstellungen gemäß Figs.1, 2a-d und 3 dargestellten, erfindungsgemäß bevorzugten Ausführungsformen.

Es zeigt:
- Fig. 1: eine schematische Darstellung eines erfindungsgemäßen Verfahrens bzw. einer erfindungsgemäßen Produktionsanlage gemäß einer bevorzugten Ausführungsform der vorliegenden Erfindung;
- Fig. 2a-d: weitere schematische Darstellungen verschiedener Varianten des erfindungsgemäßen Verfahrens bzw. der erfindungsgemäßen Produktionsanlage gemäß weiteren bevorzugten Ausführungsformen der vorliegenden Erfindung (Figs. 2b-c) im Vergleich zum Stand der Technik (Fig. 2a);
- Fig. 3: eine wiederum weitere schematische Darstellung eines erfindungsgemäßen Verfahrens bzw. einer erfindungsgemäßen Produktionsanlage gemäß einer bevorzugten Ausführungsform der vorliegenden Erfindung gemäß einer wiederum weiteren bevorzugten Ausführungsform der vorliegenden Erfindung.

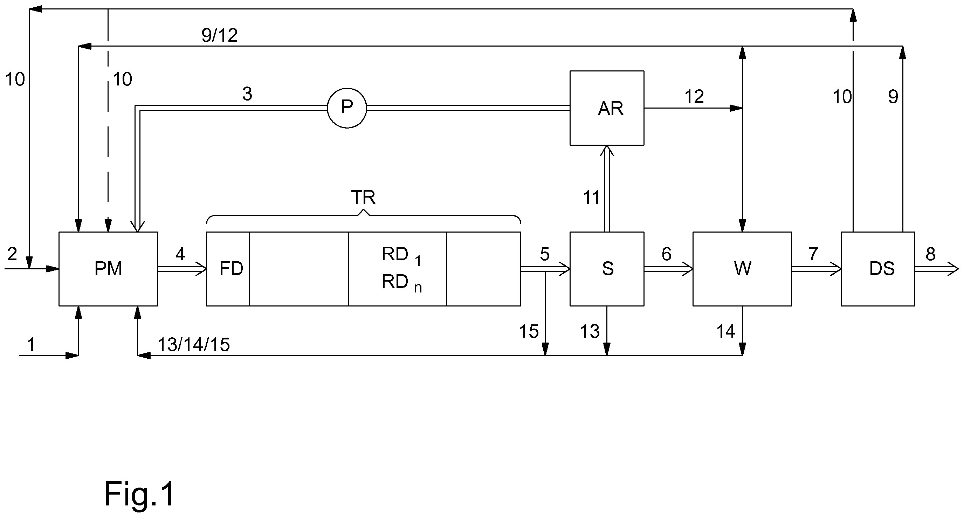

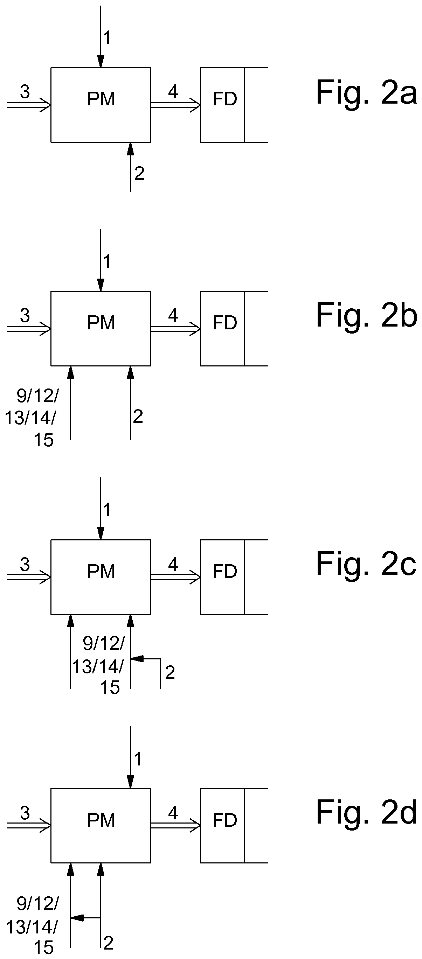

Fig. 1 zeigt in schematischer Darstellung eine Ausführungsform eines erfindungsgemäßen Verfahrens für die verschiedenen Varianten der Rückführung von Produkt in das Nitriergemisch am Beispiel der Nitrierung von Benzol zu Nitrobenzol, während in Figs. 2b-d schematisch die wichtigsten der verschiedenen möglichen Varianten und Reihenfolgen für die Zugabe der verschiedenen Edukte am Beispiel der Nitrierung von Benzol zu Nitrobenzol dargestellt werden.

Wie in Fig. 1 dargestellt, werden in einer Dosier-/Vormischeinheit PM eines Rohrreaktors TR, welche mit diesem Rohrreaktor unmittelbar verbunden ist, in die aufkonzentrierte Kreislaufschwefelsäure 3, welche durch eine geeignete Pumpe P (z. B. Kreiselpumpe) mit einem definierten Eingangsdruck P1 (mindestens die Summe aller Druckverluste im Rohrreaktor zuzüglich Enddruck P2) in die Dosier-/ Vormischeinheit PM eingespeist wird, die Edukte Salpetersäure 1, Benzol 2 und zurückgeführtes Nitrobenzol 9, 12, 13, 14 und 15 in einer vorgegebenen bzw. definierten Reihenfolge (wie in Figs. 2b-d dargestellt) eingebracht. Die Salpetersäure löst sich vollständig und homogen in der Kreislaufsäure. Das Benzol 2 und das zurückgeführte Nitrobenzol 9, 12, 13, 14 oder 15 bilden mit der Kreislaufsäure (Schwefelsäure)/Salpetersäure (= Mischsäure) ein aus zwei Phasen bestehendes Anfangsnitriergemisch 4 und werden vordispergiert. Die Temperatur in diesem Nitriergemisch 4 aus Mischsäure, Benzol und Nitrobenzol ergibt sich als Mischtemperatur (d. h. die Starttemperatur) aus den Temperaturen der einzelnen Eduktströme (1, 2, 3 und/oder 9, 12, 13, 14, 15) und wird in einem Bereich von 80 bis 120 °C derart ausgewählt, dass nach der Erstdispergierung in der Dispergiervorrichtung FD die Nitrierung anspringt. Diese in der Dosier-/Vormischeinheit PM vorgemischten Edukte werden in der Dispergiervorrichtung FD nachfolgend derart ineinander dispergiert, dass eine genügend große Austauschfläche (Phasengrenzfläche) zwischen der Organ- und Säurephase entsteht, damit die Nitrierung initiiert wird bzw. anspringt oder startet, was erkennbar ist an einem möglichst steilen Temperaturanstieg im Nitriergemisch 4 nach der Dispergiervorrichtung FD. Durch weitere im Rohrreaktor TR verteilte Dispergierelemente RD₁ bis RDₙ wird der durch Koaleszenz im Verlauf des Durchtritts des Nitriergemisches durch den Rohrrektor TR verursachten Verkleinerung der Phasengrenzfläche zwischen Organ- und Säurephase entgegengewirkt. Am Auslass des Rohrreaktors gelangt das Nitriergemisch 5 (jetzt ein Gemisch aus Endsäure und Produkt) mit einer Endtemperatur üblicherweise im Bereich von 120 bis 145 °C und mit einem Druck P2 in den Phasentrennapparat S. Der Druck P2 wird derart gewählt, dass eine Flash-Verdampfung der leichtflüchtigen Komponenten im Nitriergemisch 5, vor allem im Rohnitrobenzol (einem Gemisch aus Aliphaten/Benzol/Nitrobenzol im Fall der Nitrierung von Benzol zu Nitrobenzol), im Phasentrennapparat sicher vermieden wird.

Wie in Fig. 1 ferner gezeigt, kann nach Phasentrennung im Phasentrennapparat S ein Teilstrom 13 des Rohnitrobenzols 6, welches neben Schwefelsäure, als Mikroemulsion und gelöst, auch noch alle Verunreinigungen (wie z. B. nichtumgesetztes Benzol, Nitrophenole etc.) enthält, vor oder nach Kühlung über die Dosier-/Vormischeinheit PM in die Nitrierung zurückgeführt werden. Die Hauptmenge des Rohnitrobenzols 6 wird in der Wäsche W mit Wasser (mit und ohne Zusatz von Basen) in ein bis drei Waschschritten von allen aciden Verbindungen (z. B. Mineralsäuren, Nitrophenolen etc.) befreit. Ein Teilstrom 14 dieses Nitrobenzol/Benzol-Gemisches 7, abgezweigt aus einem oder mehreren der aus mehreren Waschschritten bestehenden Wäsche, wird bevorzugt in die Nitrierung zurückgeführt. Die Hauptmenge des Nitrobenzol/Benzol-Gemisches 7, welches bis auf Spuren frei von allen Mineralsäuren und Nitrophenolen ist, kann anschließend z. B. durch Destillation oder Dampfstrippen in der Reinigungseinheit DS vom nichtumgesetzten Benzol 10 und von leichtflüchtigen aliphatischen Verunreinigungen befreit werden. Das zurückgewonnene Benzol 10 wird nach Abtrennung der überschüssigen Aliphaten in die Nitrierung zurückgeführt. Von dem vollständig gereinigten Nitrobenzol 8 kann ebenfalls ein Teilstrom 9 in die Nitrierung zurückgeführt werden.

Wie schließlich in Fig. 1 dargestellt, können aus der in der Phasentrenneinheit S abgetrennten Endsäure 11 mit einer Temperatur von 120 bis 145 °C (z. B. durch eine Flash-Verdampfung im Verdampfer AR) das aus der Nitrierung und der Salpetersäure stammende Wasser und das in der Endsäure bis zur Löslichkeitsgrenze gelöste Nitrobenzol sowie Spuren weiterer flüchtiger Komponenten (wie z. B. Benzol, Salpetersäure, Aliphaten etc.) vollständig entfernt werden. Das bei der Flash-Verdampfung der Endsäure anfallende Brüdenkondensat, welches ein Gemisch aus Wasser und reinem Nitrobenzol 12 (welches ca. 10 % bis 15 % des insgesamt hergestellten Nitrobenzols ausmacht) ist, wird üblicherweise mit dem Rohnitrobenzol 6 aus dem Phasentrennapparat S in der Wäsche vereinigt und weiterbehandelt. Nach dem erfindungsgemäßen Verfahren kann dieses reine Nitrobenzol 12 aus dem Brüdenkondensat ebenfalls nach Phasentrennung vollständig in die Nitrierung zurückgeführt werden.

Zusätzlich kann Nitrobenzol aus der Nitrierung in den Nitrierkreislauf zurückgeführt werden derart, dass ein Teilstrom 15 des Nitriergemisches 5, vor Phasentrennung zurückgeführt wird.

Eine weitere, aber nicht dargestellte Variante des erfindungsgemäßen Verfahrens, besteht darin, Nitrobenzol aus der Abwasseraufbereitung, z. B. aus der Abwasserstrippung, zurückzuführen.

Zu den Figurendarstellungen gemäß Figs. 2a-d ist insbesondere Folgendes auszuführen:
In Fig. 2a ist zum Vergleich der Stand der Technik für die übliche Abfolge der Zumischung der Edukte zu der Kreislaufschwefelsäure, Salpetersäure und Benzol, dargestellt: Zu der Kreislaufsäure 3 wird zuerst die Salpetersäure 1 zugegeben und mit der Kreislaufsäure 3 homogen vermischt. Zu der resultierenden Mischsäure (Strom 3 + 1) wird das zu nitrierende Benzol 2 zugemischt und das resultierende Ausgangsnitriergemisch 4 oder die Ausgangsreaktionsmischung (Mischsäure/ Benzol) in der Dispergiereinheit FD derart ineinander dispergiert, dass eine genügend große Austauschfläche (Phasengrenzfläche) entsteht, damit die Nitrierung anspringt, erkennbar an einem möglichst steilen Temperaturanstieg im Ausgangsnitriergemisch 4 nach der Dispergiervorrichtung FD.

In Fig. 2b erfolgt erfindungsgemäß die Zugabe der Edukte Salpetersäure 1, Benzol 2 und Nitrobenzol aus den verschiedenen Quellen 9, 12, 13, 14 oder 15 zu der Kreislaufschwefelsäure, und zwar in der Reihenfolge Nitrobenzol aus der Quelle 9, 12, 13, 14 oder 15, dann Salpetersäure 1 und zum Schluss Benzol 2. Das Nitrobenzol kann vor Zugabe der Salpetersäure in der Kreislaufschwefelsäure 3 vordispergiert werden. Die Salpetersäure wird so schnell als möglich homogen eingemischt, und nach Zugabe des Benzols zu dem Gemisch Mischsäure/Nitrobenzol wird das Ausgangsnitriergemisch 4 in der Dispergiervorrichtung FD derart ineinander dispergiert, dass eine genügend große Austauschfläche (Phasengrenzfläche) entsteht, damit die Nitrierung anspringt, was erkennbar ist an einem möglichst steilen Temperaturanstieg im Ausgangsnitriergemisch 4 nach der Dispergiervorrichtung FD.

In Fig. 2c erfolgt die Zugabe der Edukte, Salpetersäure 1, Benzol 2 und Nitrobenzol aus den verschiedenen Quellen 9, 12, 13, 14 oder 15 zu der Kreislaufschwefelsäure 3 in der Reihenfolge Nitrobenzol aus der Quelle 9, 12, 13, 14 oder 15, Salpetersäure 1 und zum Schluss Benzol 2, welches als ein Gemisch von Benzol mit Nitrobenzol aus der Quelle 9, 12, 13, 14 oder 15 vorliegt. Das Nitrobenzol kann vor Zugabe der Salpetersäure in der Kreislaufsäure 3 vordispergiert werden. Die Salpetersäure wird so schnell als möglich homogen eingemischt, und nach Zugabe des Benzol/Nitrobenzol-Gemisches zu dem Gemisch aus Mischsäure (Nitriersäure)/Nitrobenzol wird das Ausgangsnitriergemisch 4 in der Dispergiervorrichtung FD derart ineinander dispergiert, dass eine genügend große Austauschfläche (Phasengrenzfläche) entsteht, damit die Nitrierung anspringt, was erkennbar ist an einem möglichst steilen Temperaturanstieg im Ausgangsnitriergemisch 4 nach der Dispergiervorrichtung FD.

In Fig. 2d schließlich erfolgt die Zugabe der Edukte, d. h. Salpetersäure 1, Benzol 2 und Nitrobenzol aus den verschiedenen Quellen 9, 12, 13, 14 oder 15 zu der Kreislaufschwefelsäure in der folgenden Reihenfolge: Zugabe von Nitrobenzol aus der Quelle 9, 12, 13, 14 oder 15, dann Benzol 2 allein und/oder als Gemisch mit Nitrobenzol aus der Quelle 9, 12, 13, 14 oder 15 oder in getrennten Strömen zu der Kreislaufsäure. Das Nitrobenzol/Benzol-Gemisch wird vor Zugabe der Salpetersäure in der Kreislaufsäure vordispergiert. Die Salpetersäure wird zum Schluss zugesetzt und so schnell als möglich homogen eingemischt. Dieses Ausgangsnitriergemisch wird in der Dispergiervorrichtung FD derart ineinander dispergiert, dass eine genügend große Austauschfläche (Phasengrenzfläche) entsteht, damit die Nitrierung anspringt bzw. startet, was erkennbar ist an einem möglichst steilen Temperaturanstieg im Ausgangsnitriergemisch 4 nach der Dispergiervorrichtung FD.

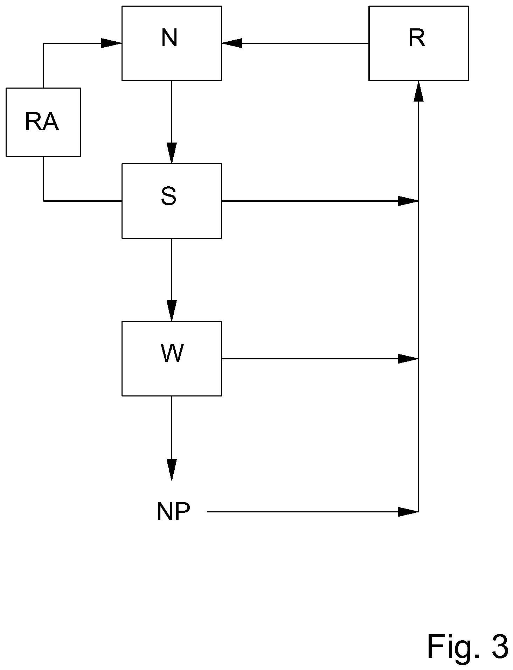

Fig. 3 zeigt eine weitere schematische Darstellung des erfindungsgemäßen Verfahrens bzw. der erfindungsgemäßen Produktionsanlage nach der vorliegenden Erfindung gemäß einer besonderen Ausführungsform der vorliegenden Erfindung: Gemäß Fig. 3 erfolgt in einer Nitriereinheit N zunächst die Nitrierung unter adiabatischen Bedingungen der nitrierbaren aromatischen organischen Ausgangsverbindungen (Aromaten) zu den entsprechenden nitrierten aromatischen organischen Verbindungen (Nitroaromaten) gemäß dem zuvor beschriebenen Reaktionsansatz (d. h. Ausgangsreaktionsmischung aus zu nitrierenden Aromaten und Salpetersäure/Schwefelsäure-Nitriersäuregemisch sowie Zusatz von entsprechendem nitriertem Produkt). In Produktionslinie stromabwärts zur Nitriereinheit N angeordnet, findet sich eine Abtrenneinrichtung S, insbesondere eine Scheideeinrichtung (Scheider), zur Abtrennung der Nitrierendsäure bzw. des Nitrierendsäuregemisches von den nitrierten Rohprodukten. In Produktionslinie stromabwärts zur Nitriereinheit N und zur Abtrenneinrichtung S angeordnet, findet sich eine Wascheinrichtung W zur Durchführung einer Wäsche der nitrierten Rohprodukte, wie zuvor beschrieben, so dass nachfolgend (nach Abtrennung des Waschmediums und gegebenenfalls Trocknung der gewaschenen nitrierten Produkte) die gewaschenen und aufgereinigten nitrierten Produkte NP entstehen.

Die erfindungsgemäße Anlage und Verfahrensführung gemäß Fig. 3 zeichnet sich - wie zuvor beschrieben - dadurch aus, dass zusätzlich eine Rückführvorrichtung R zur teilweisen Rückführung von nitriertem Produkt in die Ausgangsreaktionsmischung vorgesehen ist, was die zuvor beschriebene erfindungsgemäße Verfahrensführung ermöglicht. Dabei ist die erfindungsgemäße Rückführvorrichtung derart ausgebildet, dass das nitrierte Produkt auf verschiedensten Ebenen der erfindungsgemäßen Produktionsanlage bzw. auf verschiedensten Ebenen des erfindungsgemäßen Verfahrens entnommen und zurückgeführt werden kann, wie zuvor im Detail beschrieben (z. B. als zweiphasiges Nitriergemisch unmittelbar nach der Nitrierung und/oder aber als nitriertes Rohprodukt nach Abtrennung der Nitrierendsäure und/oder vor, aus oder nach der Wäsche, insbesondere nach der sauren oder nach der neutralen Wäsche, und/oder als reines Nitrobenzol aus der Aufkonzentrierung der Nitrierendsäure in die Rezyklierungseinheit RA oder als aufgereinigtes und gegebenenfalls getrocknetes nitriertes Endprodukt, wobei auch Kombinationen dieser Möglichkeiten vorgesehen sein können).

Die teilweise Rückführung des nitrierten Produkts zu der Ausgangsreaktionsmischung ist mit den zuvor im Detail beschriebenen Vorteilen verbunden, insbesondere mit der Verbesserung der Dispergierbarkeit von Organphase und Säurephase und damit mit einer verbesserten Reaktionsführung insgesamt (d. h. verbesserte Ausbeuten, Reduktion der Nebenproduktbildung, geringere Starttemperaturen, verbesserte Energieeffizienz, verbesserte Handhabbarkeit etc.).

Wie in Fig. 3 gezeigt und zuvor bereits ausgeführt, kann es gemäß einer weiteren besonderen Ausführungsform der erfindungsgemäßen Produktionsanlage vorgesehen sein, dass die Produktionsanlage außerdem mindestens eine Rezykliervorrichtung RA zur Rezyklierung der Nitrierendsäure umfasst. Insbesondere kann, wie zuvor erläutert, bei dieser besonderen Ausführungsform die Rezykliervorrichtung RA eine Einrichtung zur Aufkonzentrierung der Nitrierendsäure und gegebenenfalls eine Einrichtung zum Zusetzen von frischer Salpeter- und/oder Schwefelsäure umfassen.

Insgesamt werden daher im Rahmen der vorliegenden Erfindung ein verbessertes Nitrierverfahren für nitrierbare aromatische organische Verbindungen sowie eine entsprechende (Produktions-)Anlage zur Durchführung dieses Verfahrens bereitgestellt, welche sich durch eine insgesamt verbesserte Effizienz, insbesondere eine verbesserte technische Effizienz sowie eine verbesserte Energieeffizienz, und eine insgesamt verbesserte Verfahrensökonomie und zudem eine verbesserte Handhabbarkeit auszeichnen.

Weitere Ausgestaltungen, Abwandlungen, Variationen, Modifikationen oder dergleichen der vorliegenden Erfindung sind für den Fachmann beim Lesen der Beschreibung ohne Weiteres erkennbar und realisierbar, ohne dass der Rahmen der vorliegenden Erfindung verlassen wird.

Die vorliegende Erfindung wird anhand der folgenden Ausführungsbeispiele veranschaulicht, ohne jedoch die vorliegende Erfindung hierauf zu beschränken.

### AUSFÜHRUNGSBEISPIELE:

### Beispiel 1 (Vergleichsbeispiel)

In einen Rohrreaktor mit einem Innenvolumen von 231 ml, welcher mit 17 Mischelementen (Kombination aus statischen Mischelementen und Blenden) in ungleichmäßigen Abständen bestückt ist, werden für eine Starttemperatur von 80 °C 762 g/h Benzol (10 % Überschuss) und 15,4 kg/h Mischsäure mit einem Gehalt an Schwefelsäure von 65,73 % und an Salpetersäure von 4,99 % dosiert. Das Benzol wird zur Erstdispergierung über eine Düse in den Reaktor bei ca. 9 bar Innendruck eingebracht. Bei einer Verweilzeit des Nitriergemischs (mit einem Endsäure/Produkt-Phasenverhältnis von 11,7 gewichtsbasiert bzw. von 9,6 volumenbasiert) im Reaktor von 80 s liegt die Fließgeschwindigkeit des Nitriergemisches bei 0,42 m/s. Die Temperatur im Reaktor beträgt nach dem fünften Mischelement nach der Erstdispergierung 98 °C und am Ende des Reaktors 121 °C Der Restgehalt an Salpetersäure im Nitrierendsäure liegt bei 1.200 ppm (entsprechend einem Umsatz an Salpetersäure von 97 %). Der Temperaturanstieg im Nitriergemisch liegt bei 41 °C. Das Rohnitrobenzol enthält - neben dem Überschuss an dosiertem Benzol - weniger als 120 ppm an Dinitrobenzol (DNB) und weniger als 1.200 ppm an Nitrophenolen mit einem sehr kleinen Anteil an Pikrinsäure.

### Beispiel 2 (erfindungsgemäß)

In einen Rohrreaktor, wie in Beispiel 1 beschrieben, wird für eine Starttemperatur von 80 °C ein Gemisch aus 762 g/h Benzol (10 % Überschuss) und 190,5 g/h rückgeführtes, aus der Nitrierung stammendes Nitrobenzol (entsprechend 25 % des dosierten Benzols) sowie 15,4 kg/h Mischsäure mit einem Gehalt an Schwefelsäure von 65,73 % und an Salpetersäure von 4,99 % dosiert. Das Benzol/Nitrobenzol-Gemisch wird zur Erstdispergierung über eine Düse in den Reaktor bei ca. 9 bar Innendruck eingebracht. Bei einer Verweilzeit des Nitriergemischs (mit einem Endsäure/Produkt-Phasenverhältnis von 10,2 gewichtsbasiert bzw. von 8,0 volumenbasiert) im Reaktor von 78 s liegt die Fließgeschwindigkeit des Nitriergemisches bei 0,43 m/s. Die Temperatur im Reaktor beträgt nach dem fünften Mischelement nach der ersten Dispergierung 102 °C und am Ende des Reaktors 122,1 °C. Der Restgehalt an Salpetersäure in der Nitrierendsäure liegt bei weniger als 200 ppm (entsprechend einem Umsatz an Salpetersäure von 99,5 %). Der Temperaturanstieg im Nitriergemisch liegt bei 42,1 °C. Das Rohnitrobenzol enthält - neben dem Überschuss an dosiertem Benzol - ca. 80 ppm an Dinitrobenzol (DNB) und weniger als 900 ppm an Nitrophenolen mit einem sehr kleinen Anteil an Pikrinsäure.

### Beispiel 3 (erfindungsgemäß)

In einen Rohrreaktor, wie in Beispiel 1 beschrieben, wird für eine Starttemperatur von ca. 80 °C ein Gemisch aus 15,4 kg/h Mischsäure mit einem Gehalt an Schwefelsäure von 65,73 % und an Salpetersäure von 4,99 % sowie 190,5 g/h rückgeführtes, aus der Nitrierung stammendes Nitrobenzol (entsprechend 1,31 % der Mischsäure) und 762 g/h Benzol (10 % Überschuss) dosiert, wobei das Nitrobenzol vor Zugabe des Benzols in die Mischsäure vordispergiert wird. Das Benzol wird zur Erstdispergierung über eine Düse in den Reaktor in das Gemisch aus Mischsäure und Nitrobenzol bei ca. 9 bar Innendruck eingebracht. Bei einer Verweilzeit des Nitriergemisch mit einem Endsäure/Produkt-Phasenverhältnis von 10,2 gewichtsbasiert bzw. von 8,0 volumenbasiert im Reaktor von 78 s liegt die Fließgeschwindigkeit des Nitriergemisches bei 0,43 m/s. Die Temperatur im Reaktor beträgt nach dem fünften Mischelement nach der ersten Vordispergierung 102 °C und am Ende des Reaktors 122,1 °C. Der Restgehalt an Salpetersäure im Nitrierendsäure liegt bei weniger als 200 ppm (entsprechend einem Umsatz an Salpetersäure von 99,5 %). Der Temperaturanstieg im Nitriergemisch liegt bei 42,1 °C. Das Rohnitrobenzol enthält neben dem Überschuss an dosiertem Benzol ca. 80 bis 90 ppm an Dinitrobenzol (DNB) und weniger als 900 ppm an Nitrophenolen mit einem sehr kleinen Anteil an Pikrinsäure.

### Beispiel 4 (erfindunasaemäß)

In einen Rohrreaktor, wie in Beispiel 1 beschrieben, wird für eine Starttemperatur von ca. 80 °C ein Gemisch aus 15,4 kg/h Mischsäure mit einem Gehalt an Schwefelsäure von 65,73 % und an Salpetersäure von 4,99 % und 160 g/h Nitrobenzol (entsprechend 1,1 % der Kreislaufsäure) sowie ein Gemisch aus 762 g/h Benzol (10 % Überschuss) und 76 g/h Nitrobenzol (entsprechend 10 % des dosierten Benzols) dosiert, wobei das Nitrobenzol vor Zugabe des Benzols in der Mischsäure vordispergiert wird. Das Benzol/Nitrobenzol-Gemisch wird zur Erstdispergierung über eine Düse in den Reaktor in das Gemisch aus Mischsäure und Nitrobenzol bei ca. 9 bar Innendruck eingebracht. Bei einer Verweilzeit des Nitriergemisches mit einem Endsäure/Produkt-Phasenverhältnis von 10,2 gewichtsbasiert bzw. von 8,0 volumenbasiert im Reaktor von 78 s liegt die Fließgeschwindigkeit des Nitriergemisches bei ca. 0,43 m/s. Die Temperatur im Reaktor beträgt nach dem fünften Mischelement nach der ersten Vordispergierung 102 °C und am Ende des Reaktors 122,1 °C. Der Restgehalt an Salpetersäure in der Nitrierendsäure liegt bei weniger als 200 ppm (entsprechend einem Umsatz an Salpetersäure von 99,5 %). Der Temperaturanstieg im Nitriergemisch liegt bei 42,1 °C. Das Rohnitrobenzol enthält neben dem Überschuss an dosiertem Benzol ca. 80 ppm an Dinitrobenzol (DNB) und weniger 900 ppm an Nitrophenolen mit einem sehr kleinen Anteil an Pikrinsäure.

## Patentansprüche

1. Verfahren zur adiabatischen Nitrierung von nitrierbaren aromatischen organischen Verbindungen (Aromaten) zu den entsprechenden nitrierten aromatischen organischen Verbindungen (Nitroaromaten),
wobei nitrierbare aromatische organische Verbindungen (Aromaten) in einer Nitrierreaktion mit einem Salpetersäure/Schwefelsäure-Nitriersäuregemisch zu den entsprechenden nitrierten aromatischen organischen Verbindungen (Nitroaromaten) umgesetzt werden,
wobei der Ausgangsreaktionsmischung, welche die nitrierbaren aromatischen organischen Verbindungen (Aromaten) und das Salpetersäure/Schwefelsäure-Nitriersäuregemisch umfasst, entsprechende nitrierte aromatische organische Verbindungen (Nitroaromaten) zugesetzt werden und die Umsetzung und/oder Nitrierreaktion in Gegenwart der nitrierten aromatischen organischen Verbindungen (Nitroaromaten) gestartet und/oder durchgeführt wird; und
wobei die erhaltenen nitrierten aromatischen organischen Verbindungen (Nitroaromaten) teilweise in die Nitrierreaktion zurückgeführt werden und die nachfolgende Umsetzung und/oder Nitrierreaktion in Gegenwart der nitrierten aromatischen organischen Verbindungen (Nitroaromaten) gestartet und/oder durchgeführt wird;
wobei die Umsetzung und/oder Nitrierreaktion unter adiabatischen Reaktionsbedingungen durchgeführt wird,
wobei die Umsetzung und/oder Nitrierreaktion in einem Rohrreaktor durchgeführt wird und
wobei die für die Umsetzung und/oder Nitrierreaktion zugesetzte und/oder zurückgeführte Menge an nitrierten aromatischen organischen Verbindungen (Nitroaromaten) derart ausgewählt wird, dass die Menge an zugesetzten und/oder zurückgeführten nitrierten aromatischen organischen Verbindungen (Nitroaromaten) eine Absenkung der Grenzflächenspannung zwischen Organphase und Säurephase bewirkt und eine verbesserte Dispergierbarkeit von Organphase und Säurephase bewirkt.

2. Verfahren nach Anspruch 1,
wobei die nach der Umsetzung und/oder Nitrierreaktion resultierende Nitrierendsäure nach Abtrennung der rohen nitrierten aromatischen organischen Verbindungen (Roh-Nitroaromaten) rezykliert wird und/oder im Kreislauf gefahren wird und/oder wieder in die Nitrierreaktion zurückgeführt wird, insbesondere nach Aufkonzentrierung und/oder nach Zusetzen von frischer Salpeter- und/oder Schwefelsäure.

3. Verfahren nach einem der vorangehenden Ansprüche, wobei die für die Umsetzung und/oder Nitrierreaktion zugesetzte und/oder zurückgeführte Menge an nitrierten aromatischen organischen Verbindungen (Nitroaromaten) derart ausgewählt wird,
dass die gewichtsbezogene Menge an zugesetzten und/oder zurückgeführten nitrierten aromatischen organischen Verbindungen (Nitroaromaten), bezogen auf die zu nitrierenden und/oder umzusetzenden nitrierbaren aromatischen organischen Verbindungen (Aromaten), im Bereich von 0,01 bis 60 Gew.-%, insbesondere im Bereich von 0,1 bis 50 Gew.-%, vorzugsweise im Bereich von 5 bis 45 Gew.-%, besonders bevorzugt im Bereich von 10 bis 40 Gew.-%, liegt; und/oder
dass die gewichtsbezogene Menge an zugesetzten und/oder zurückgeführten nitrierten aromatischen organischen Verbindungen (Nitroaromaten), bezogen auf die Schwefelsäure des Salpetersäure/Schwefelsäure-Nitriersäuregemisches, im Bereich von 0,01 bis 10 Gew.-%, insbesondere im Bereich von 0,2 bis 5 Gew.-%, vorzugsweise im Bereich von 0,5 bis 3 Gew.-%, besonders bevorzugt im Bereich von 1 bis 2 Gew.-%, liegt.

4. Verfahren nach einem der vorangehenden Ansprüche,
wobei die für die Umsetzung und/oder Nitrierreaktion zugesetzten und/oder zurückgeführten nitrierten aromatischen organischen Verbindungen (Nitroaromaten) mindestens einer der nachfolgenden Positionen (i) bis (iv) zugesetzt und/oder zugeführt werden: (i) der Ausgangsreaktionsmischung aller übrigen Reaktanden; und/oder (ii) der Schwefelsäure des Salpetersäure/ Schwefelsäure-Nitriersäuregemisches, insbesondere vor Herstellung des Salpetersäure/Schwefelsäure-Nitriersäuregemisches; und/oder (iii) dem Salpetersäure/Schwefelsäure-Nitriersäuregemisch; und/oder (iv) den zu nitrierenden nitrierbaren aromatischen organischen Verbindungen (Aromaten).

5. Verfahren nach einem der vorangehenden Ansprüche,
wobei die für die Umsetzung und/oder Nitrierreaktion zugesetzten und/oder zurückgeführten nitrierten aromatischen organischen Verbindungen (Nitroaromaten) mindestens aus einer der nachfolgenden Positionen (i) bis (iv) stammen: (i) den rohen nitrierten aromatischen organischen Verbindungen (Roh-Nitroaromaten), vorzugsweise nach Abtrennung der sauren wässrigen Phase (Säurephase) und/oder nach Phasentrennung des erhaltenen Nitriergemisches in Nitrierendsäure und rohe nitrierte aromatische organische Verbindungen (Roh-Nitroaromaten); und/oder (ii) den gewaschenen nitrierten aromatischen organischen Verbindungen (Nitroaromaten), insbesondere nach der sauren oder neutralen Wäsche; und/oder (iii) den gewaschenen, insbesondere nach der sauren oder neutralen Wäsche erhaltenen, sowie gestrippten oder destillierten oder getrockneten nitrierten aromatischen organischen Verbindungen (Nitroaromaten); und/oder (iv) den nach der Aufkonzentrierung der Nitrierendsäure im Brüdenkondensat anfallenden nitrierten aromatischen organischen Verbindungen (Nitroaromaten).

6. Verfahren nach einem der vorangehenden Ansprüche,
wobei die für die Umsetzung und/oder Nitrierreaktion zugesetzten und/oder zurückgeführten nitrierten aromatischen organischen Verbindungen (Nitroaromaten) sowohl der Organphase als auch der Säurephase der Ausgangsreaktionsmischung zugeführt und/oder zugesetzt werden;
insbesondere wobei der Organphase, bezogen auf die Organphase, 0,1 bis 35 Gew.-%, insbesondere 10 bis 25 Gew.-%, nitrierte aromatische organische Verbindungen (Nitroaromaten) zugesetzt werden und/oder insbesondere wobei der Säurephase, bezogen auf die Säurephase, 0,01 bis 3 Gew.-%, insbesondere 0,1 bis 2 Gew.-%, vorzugsweise 0,5 bis 1,5 Gew.-%, besonders bevorzugt 1,1 bis 1,5 Gew.-%, nitrierte aromatische organische Verbindungen (Nitroaromaten) zugesetzt werden.

7. Verfahren nach einem der vorangehenden Ansprüche,
wobei die nach der Umsetzung und/oder Nitrierreaktion resultierende Nitrierendsäure nach Abtrennung der rohen nitrierten aromatischen organischen Verbindungen (Roh-Nitroaromaten) und nach anschließender Aufkonzentrierung und gegebenenfalls Zusetzen von frischer Salpeter- und/oder Schwefelsäure rezykliert wird und/oder im Kreislauf gefahren wird und/oder wieder in die Nitrierreaktion zurückgeführt wird, wobei die für die Umsetzung und/oder Nitrierreaktion zugesetzten und/oder zurückgeführten nitrierten aromatischen organischen Verbindungen (Nitroaromaten) der aufkonzentrierten und gegebenenfalls mit frischer Salpeter- und/oder Schwefelsäure versetzten Nitrierendsäure und/oder den zu nitrierenden nitrierbaren aromatischen organischen Verbindungen (Aromaten) zugesetzt und/oder zugeführt werden;
insbesondere wobei die nitrierbaren aromatischen organischen Verbindungen (Aromaten) vorzugsweise unmittelbar vor Reaktionsbeginn und/oder als zeitlich letzte Reaktionskomponente (Reaktand) zugesetzt und/oder zugeführt werden, vorzugsweise unmittelbar vor der die Umsetzung und/oder Nitrierreaktion auslösenden Erstdispergierung.

8. Verfahren nach einem der vorangehenden Ansprüche,
wobei die nach der Umsetzung und/oder Nitrierreaktion resultierende Nitrierendsäure nach Abtrennung der rohen nitrierten aromatischen organischen Verbindungen (Roh-Nitroaromaten) und nach anschließender Aufkonzentrierung als Kreislaufsäure rezykliert wird und/oder im Kreislauf gefahren wird und/oder wieder in die Nitrierreaktion zurückgeführt wird, wobei zunächst eine Dispersion aus aufkonzentrierter Kreislaufsäure und zu nitrierenden nitrierbaren aromatischen organischen Verbindungen (Aromaten) sowie nitrierten aromatischen organischen Verbindungen (Nitroaromaten) erzeugt wird, wobei der Dispersion anschließend Salpetersäure zugesetzt, insbesondere zudispergiert, wird und auf diese Weise die Nitrierreaktion initiiert wird;
insbesondere wobei die Salpetersäure vorzugsweise unmittelbar vor Reaktionsbeginn und/oder als zeitlich letzte Reaktionskomponente (Reaktand) zugesetzt und/oder zugeführt wird, vorzugsweise unmittelbar vor der die Umsetzung und/oder Nitrierreaktion auslösenden Erstdispergierung.

9. Produktionsanlage (Nitrieranlage bzw. Anlage) zur adiabatischen Nitrierung von nitrierbaren aromatischen organischen Verbindungen (Aromaten) zu nitrierten Produkten in Form der entsprechenden nitrierten aromatischen organischen Verbindungen (Nitroaromaten), insbesondere Produktionsanlage zur Durchführung eines Verfahrens gemäß einem der vorangehenden Ansprüche,
wobei die Produktionsanlage die folgenden Einheiten und Vorrichtungen umfasst:
(a) eine Nitriereinheit (N) zur Nitrierung, insbesondere adiabatischen Nitrierung, von nitrierbaren aromatischen organischen Verbindungen (Aromaten) zu nitrierten Produkten in Form der entsprechenden nitrierten aromatischen organischen Verbindungen (Nitroaromaten), insbesondere mit einem oder mehreren Reaktoren zur Durchführung der Nitrierreaktion, wobei die Nitriereinheit (N) als Reaktor mindestens einen Rohrreaktor umfasst;
(b) in Produktionslinie stromabwärts zur Nitriereinheit (N) angeordnet, mindestens eine Abtrennvorrichtung (S), insbesondere eine Scheidevorrichtung (Scheider), zur Abtrennung der Nitrierendsäure von den nitrierten Rohprodukten;
(c) in Produktionslinie stromabwärts zur Nitriereinheit (N) und zur Abtrennvorrichtung (S) angeordnet, mindestens eine Wascheinheit (W) zur Durchführung einer Wäsche der nitrierten Rohprodukte mit einem Waschmedium, insbesondere in einem oder mehreren Waschschritten;
(d) in Produktionslinie stromabwärts zur Wascheinheit (W) angeordnet, eine Abtrennvorrichtung, insbesondere eine Scheidevorrichtung (Scheider), zur Abtrennung der gewaschenen nitrierten Produkte vom Waschmedium;
wobei die Produktionsanlage außerdem mindestens eine Rückführvorrichtung (R) zur teilweisen Rückführung der nitrierten Produkte in die Nitriereinheit (N), insbesondere in die Ausgangsreaktionsmischung der Nitriereinheit (N), umfasst.

10. Produktionsanlage nach Anspruch 9,
wobei der Rohrreaktor der Nitriereinheit (N) mit einem oder mehreren, vorzugsweise mit mehreren Mischelementen, insbesondere zum Eintrag von zusätzlicher Mischenergie, ausgestattet ist;
insbesondere wobei die Mischelemente als Bleche, insbesondere Prall- oder Umlenkbleche, als Blenden, als statische Mischer oder als Stromteiler ausgebildet sind; und/oder
insbesondere wobei die Mischelemente derart ausgebildet sind, dass durch die Mischelemente im Betriebszustand eine Mischenergie von 10 bis 1000 Joule/Liter, bevorzugt 10 bis 500 Joule/Liter, besonders bevorzugt 20 bis 200 Joule/Liter, eingetragen wird; und/oder
insbesondere wobei im Betriebszustand der Druckabfall pro Mischelement 0,1 bar bis 3,0 bar, bevorzugt 0,3 bis 1,5 bar, besonders bevorzugt 0,3 bis 0,8 bar, beträgt; und/oder
insbesondere wobei die Mischelemente derart im Rohrreaktor angeordnet sind, dass im Betriebszustand in den ersten 10 bis 30 Vol.-% des Reaktors der Umsatz der Salpetersäure des Salpetersäure/Schwefelsäure-Nitriersäuregemisches mindestens 40 %, insbesondere mindestens 50 %, vorzugsweise mindestens 60 %, beträgt; und/oder
insbesondere wobei die Mischelemente derart im Rohrreaktor angeordnet sind, dass der Umsatz an eingesetzter Salpetersäure am Ende des Rohrreaktors mindestens 98 %, bevorzugt mindestens 99 %, besonders bevorzugt mindestens 99,5 %, beträgt.

11. Produktionsanlage nach einem der vorangehenden Ansprüche,
wobei die Rückführvorrichtung (R) zur teilweisen Rückführung der nitrierten Produkte in die Nitriereinheit (N) derart ausgebildet und/oder angeordnet ist, dass die teilweise rückzuführenden nitrierten Produkte mindestens aus einer der nachfolgenden Positionen (i) bis (iv) des Produktionsstroms entnommen werden: (i) den rohen nitrierten aromatischen organischen Verbindungen (Roh-Nitroaromaten), vorzugsweise nach Abtrennung der sauren wässrigen Phase (Säurephase) und/oder nach Phasentrennung des erhaltenen Nitriergemisches in Nitrierendsäure und rohe nitrierte aromatische organische Verbindungen (Roh-Nitroaromaten); und/oder (ii) den gewaschenen nitrierten aromatischen organischen Verbindungen (Nitroaromaten), insbesondere nach der sauren oder neutralen Wäsche; und/oder (iii) den gewaschenen, insbesondere nach der sauren oder neutralen Wäsche erhaltenen, sowie gestrippten oder destillierten oder getrockneten nitrierten aromatischen organischen Verbindungen (Nitroaromaten); und/oder (iv) den nach der Aufkonzentrierung der Nitrierendsäure im Brüdenkondensat anfallenden nitrierten aromatischen organischen Verbindungen (Nitroaromaten).

12. Produktionsanlage nach einem der vorangehenden Ansprüche,
wobei die Rückführvorrichtung (R) zur teilweisen Rückführung der nitrierten Produkte in die Nitriereinheit (N) derart ausgebildet und/oder angeordnet ist, dass die teilweise rückzuführenden nitrierten Produkte mindestens einer der nachfolgenden Positionen (i) bis (iv) des Produktionsstroms zugesetzt und/oder zugeführt werden: (i) der Ausgangsreaktionsmischung aller übrigen Reaktanden; und/oder (ii) der Schwefelsäure des Salpetersäure/Schwefelsäure-Nitriersäuregemisches, insbesondere vor Herstellung des Salpetersäure/Schwefelsäure-Nitriersäuregemisches; und/oder (iii) dem Salpetersäure/Schwefelsäure-Nitriersäuregemisch; und/oder (iv) den zu nitrierenden nitrierbaren aromatischen organischen Verbindungen (Aromaten).

13. Produktionsanlage nach einem der vorangehenden Ansprüche,
wobei die Produktionsanlage außerdem mindestens eine Rezykliervorrichtung (RA) zur Rezyklierung der Nitrierendsäure umfasst;
insbesondere wobei die Rezykliervorrichtung (RA) eine Einrichtung zur Aufkonzentrierung der Nitrierendsäure und gegebenenfalls eine Einrichtung zum Zusetzen von frischer Salpeter- und/oder Schwefelsäure umfasst.

14. Verwendung von nitrierten aromatischen organischen Verbindungen (Nitroaromaten) zur Erniedrigung der Grenzflächenspannung von Organphase und Säurephase und/oder zur Verbesserung der Dispergierbarkeit von Organphase und Säurephase bei Nitrierungen, insbesondere bei Nitrierreaktionen der entsprechenden unnitrierten aromatischen organischen Verbindungen,
wobei nitrierbare aromatische organische Verbindungen (Aromaten) in einer adiabatischen Nitrierreaktion mit einem Salpetersäure/Schwefelsäure-Nitriersäuregemisch zu den entsprechenden nitrierten aromatischen organischen Verbindungen (Nitroaromaten) umgesetzt werden;
wobei der Ausgangsreaktionsmischung, welche die nitrierbaren aromatischen organischen Verbindungen (Aromaten) und das Salpetersäure/Schwefelsäure-Nitriersäuregemisch umfasst, entsprechende nitrierte aromatische organische Verbindungen (Nitroaromaten) zugesetzt werden und die Umsetzung und/oder Nitrierreaktion in Gegenwart der nitrierten aromatischen organischen Verbindungen (Nitroaromaten) gestartet und/oder durchgeführt wird; und
wobei die erhaltenen nitrierten aromatischen organischen Verbindungen (Nitroaromaten) teilweise in die Nitrierreaktion zurückgeführt werden und die nachfolgende Umsetzung und/oder Nitrierreaktion in Gegenwart der nitrierten aromatischen organischen Verbindungen (Nitroaromaten) gestartet und/oder durchgeführt wird;
wobei die Umsetzung und/oder Nitrierreaktion unter adiabatischen Reaktionsbedingungen durchgeführt wird,
wobei die Umsetzung und/oder Nitrierreaktion in einem Rohrreaktor durchgeführt wird und
wobei die für die Umsetzung und/oder Nitrierreaktion zugesetzte und/oder zurückgeführte Menge an nitrierten aromatischen organischen Verbindungen (Nitroaromaten) derart ausgewählt wird, dass die Menge an zugesetzten und/oder zurückgeführten nitrierten aromatischen organischen Verbindungen (Nitroaromaten) eine Absenkung der Grenzflächenspannung zwischen Organphase und Säurephase bewirkt und eine verbesserte Dispergierbarkeit von Organphase und Säurephase bewirkt.

15. Verwendung von nitrierten aromatischen organischen Verbindungen (Nitroaromaten) zur Erhöhung der Ausbeuten und/oder zur Reduktion der Nebenproduktbildung und/oder zur Verkürzung des Reaktionsgesamtdauern und/oder zur Erniedrigung der Reaktionsstarttemperaturen bei Nitrierungen, insbesondere bei Nitrierreaktionen der entsprechenden unnitrierten aromatischen organischen Verbindungen,
wobei nitrierbare aromatische organische Verbindungen (Aromaten) in einer adiabatischen Nitrierreaktion mit einem Salpetersäure/Schwefelsäure-Nitriersäuregemisch zu den entsprechenden nitrierten aromatischen organischen Verbindungen (Nitroaromaten) umgesetzt werden;
wobei der Ausgangsreaktionsmischung, welche die nitrierbaren aromatischen organischen Verbindungen (Aromaten) und das Salpetersäure/Schwefelsäure-Nitriersäuregemisch umfasst, entsprechende nitrierte aromatische organische Verbindungen (Nitroaromaten) zugesetzt werden und die Umsetzung und/oder Nitrierreaktion in Gegenwart der nitrierten aromatischen organischen Verbindungen (Nitroaromaten) gestartet und/oder durchgeführt wird; und
wobei die erhaltenen nitrierten aromatischen organischen Verbindungen (Nitroaromaten) teilweise in die Nitrierreaktion zurückgeführt werden und die nachfolgende Umsetzung und/oder Nitrierreaktion in Gegenwart der nitrierten aromatischen organischen Verbindungen (Nitroaromaten) gestartet und/oder durchgeführt wird;
wobei die Umsetzung und/oder Nitrierreaktion unter adiabatischen Reaktionsbedingungen durchgeführt wird,
wobei die Umsetzung und/oder Nitrierreaktion in einem Rohrreaktor durchgeführt wird und
wobei die für die Umsetzung und/oder Nitrierreaktion zugesetzte und/oder zurückgeführte Menge an nitrierten aromatischen organischen Verbindungen (Nitroaromaten) derart ausgewählt wird, dass die Menge an zugesetzten und/oder zurückgeführten nitrierten aromatischen organischen Verbindungen (Nitroaromaten) eine Absenkung der Grenzflächenspannung zwischen Organphase und Säurephase bewirkt und eine verbesserte Dispergierbarkeit von Organphase und Säurephase bewirkt.

## Claims

1. A method for adiabatic nitration of nitrable aromatic organic compounds (aromates) to the corresponding nitrated aromatic organic compounds (nitroaromates),
wherein nitrable aromatic organic compounds (aromates) are converted in a nitration reaction with a nitric acid/sulphuric acid nitrating acid mixture into the corresponding nitrated aromatic organic compounds (nitroaromates),
wherein corresponding nitrated aromatic organic compounds (nitroaromates) are added to the initial reaction mixture, which comprises the nitrable aromatic organic compounds (aromates) and the nitric acid/sulphuric acid nitrating acid mixture, and the conversion and/or nitration reaction is started and/or carried out in the presence of the nitrated aromatic organic compounds (nitroaromates); and
wherein the nitrated aromatic organic compounds (nitroaromates) obtained are partially returned to the nitration reaction and the subsequent conversion and/or nitration reaction is started and/or carried out in the presence of the nitrated aromatic organic compounds (nitroaromates);
wherein the conversion and/or nitration reaction is carried out under adiabatic reaction conditions,
wherein the conversion and/or nitration reaction is carried out in a tubular reactor, and
wherein the amount of nitrated aromatic organic compounds (nitroaromates) added and/or returned for the conversion and/or nitration reaction is selected such that the amount of nitrated aromatic organic compounds (nitroaromates) added and/or returned reduces the interfacial tension between the organic phase and acid phase and improves dispersibility of the organic phase and acid phase.

2. The method according to claim 1,
wherein the nitrating acid resulting after the conversion and/or nitration reaction is recycled after the crude nitrated aromatic organic compounds (crude nitroaromates) have been separated off and/or is circulated and/or is returned to the nitration reaction, in particular after concentration and/or after adding fresh nitric and/or sulphuric acid.

3. The method according to any one of the preceding claims, wherein the amount of nitrated aromatic organic compounds (nitroaromates) added and/or returned for the conversion and/or nitration reaction is selected in such a way
that the weight-based amount of added and/or returned nitrated aromatic organic compounds (nitroaromates), based on the nitrable aromatic organic compounds (aromates) to be nitrated and/or converted, is in the range from 0.01 to 60% by weight, in particular in the range from 0.1 to 50% by weight, preferably in the range from 5 to 45% by weight, particularly preferably in the range from 10 to 40% by weight; and/or
that the weight-based amount of added and/or returned nitrated aromatic organic compounds (nitroaromates), based on the sulphuric acid of the nitric acid/sulphuric acid nitrating acid mixture, is in the range from 0.01 to 10% by weight, in particular in the range from 0.2 to 5% by weight, preferably in the range from 0.5 to 3% by weight, particularly preferably in the range from 1 to 2% by weight.

4. The method according to any one of the preceding claims,
wherein the nitrated aromatic organic compounds (nitroaromates) added and/or returned for the conversion and/or nitration reaction are added and/or supplied to at least one of the following positions (i) to (iv): (i) the initial reaction mixture of all other reactants; and/or (ii) the sulphuric acid of the nitric acid/sulphuric acid nitrating acid mixture, in particular before the preparation of the nitric acid/sulphuric acid nitrating acid mixture; and/or (iii) the nitric acid/sulphuric acid nitrating acid mixture; and/or (iv) the nitrable aromatic organic compounds (aromates) to be nitrated.

5. The method according to any one of the preceding claims,
wherein the nitrated aromatic organic compounds (nitroaromates) added and/or returned for the conversion and/or nitration reaction originate at least from one of the following positions (i) to (iv): (i) the crude nitrated aromatic organic compounds (crude nitroaromates), preferably after separation of the acidic aqueous phase (acid phase) and/or after phase separation of the obtained nitration mixture into nitrating acid and crude nitrated aromatic organic compounds (crude nitroaromates); and/or (ii) the washed nitrated aromatic organic compounds (nitroaromates), in particular after acidic or neutral washing; and/or (iii) the washed and stripped or distilled or dried nitrated aromatic organic compounds (nitroaromates), obtained in particular after acidic or neutral washing; and/or (iv) the nitrated aromatic organic compounds (nitroaromates) obtained after the concentration of the nitrating acid in the vapour condensate.

6. The method according to any one of the preceding claims,
wherein the nitrated aromatic organic compounds (nitroaromates) added and/or returned for the conversion and/or nitration reaction are supplied to and/or added to both the organic phase and the acid phase of the initial reaction mixture;
in particular wherein 0.1 to 35% by weight, in particular 10 to 25% by weight, nitrated aromatic organic compounds (nitroaromates) are added to the organic phase, based on the organic phase, and/or in particular 0.01 to 3% by weight, in particular 0.1 to 2% by weight, preferably 0.5 to 1.5% by weight, particularly preferably 1.1 to 1.5% by weight, nitrated aromatic organic compounds (nitroaromates) are added to the acid phase, based on the acid phase.

7. The method according to any one of the preceding claims,
wherein the nitrating acid resulting after the conversion and/or nitration reaction is recycled after the crude nitrated aromatic organic compounds (crude nitroaromates) have been separated off and after subsequent concentration and optional addition of fresh nitric and/or sulphuric acid then concentrated and optionally added, and/or is circulated and/or is returned to the nitration reaction, wherein the nitrated aromatic organic compounds (nitroaromates) added and/or returned for the conversion and/or nitration reaction are added and/or returned to the concentrated nitrating acid, optionally mixed with fresh nitric and/or sulphuric acid, and/or the nitrable aromatic organic compounds (aromates) to be nitrated;
in particular wherein the nitrable aromatic organic compounds (aromates) are preferably added and/or supplied immediately before the start of the reaction and/or as the last reaction component (reactant), preferably immediately before the initial dispersion triggering the conversion and/or nitration reaction.

8. The method according to any one of the preceding claims,
wherein the nitrating acid resulting after the conversion and/or nitration reaction is recycled as circulating acid after separation of the crude nitrated aromatic organic compounds (crude nitroaromates) and after subsequent concentration and/or is circulated and/or returned back to the nitration reaction, wherein a dispersion of concentrated circulating acid and nitrable aromatic organic compounds (aromates) to be nitrated and nitrated aromatic organic compounds (nitroaromates) is initially produced, wherein nitric acid is subsequently added to the dispersion, in particular dispersed in, and in this way the nitration reaction is initiated;
in particular wherein the nitric acid is preferably added and/or supplied immediately before the start of the reaction and/or as the last reaction component (reactant), preferably immediately before the initial dispersion triggering the conversion and/or nitration reaction.

9. A production plant (nitrating plant or system) for adiabatic nitration of nitrable aromatic organic compounds (aromates) to nitrated products in the form of the corresponding nitrated aromatic organic compounds (nitroaromates), in particular a production plant for carrying out a method according to any one of the preceding claims,
wherein the production plant comprises the following units and devices:
(a) a nitration unit (N) for nitration, in particular adiabatic nitration, from nitrable aromatic organic compounds (aromates) to nitrated products in the form of the corresponding nitrated aromatic organic compounds (nitroaromates), in particular with one or more reactors for carrying out the nitration reaction, wherein the nitration unit (N) comprises at least one tubular reactor as the reactor;
(b) arranged in the production line downstream of the nitration unit (N), at least one disconnecting device (S), in particular a separating device (separator), for separating the nitrating acid from the nitrated crude products;
(c) arranged in the production line downstream of the nitration unit (N) and the disconnecting device (S), at least one washing unit (W) for carrying out washing of the nitrated crude products with a washing medium, in particular in one or a plurality of washing steps;
(d) arranged in the production line downstream of the washing unit (W), a disconnecting device, in particular a separating device (separator), for separating the washed nitrated products from the washing medium;
wherein the production plant also comprises at least one return device (R) for partially returning the nitrated products to the nitration unit (N), in particular to the initial reaction mixture of the nitration unit (N).

10. The production plant according to claim 9,
wherein the tubular reactor of the nitration unit (N) is equipped with one or a plurality of, preferably with a plurality of, mixing elements, in particular for introducing additional mixing energy;
in particular wherein the mixing elements are designed as sheets, in particular baffle or deflection sheets, as screens, as static mixers or as flow dividers; and/or
in particular wherein the mixing elements are designed such that a mixing energy of 10 to 1000 joules/litre, preferably 10 to 500 joules/litre, particularly preferably 20 to 200 joules/litre, is introduced by the mixing elements in the operating state; and/or
in particular wherein in the operating state the pressure drop per mixing element is 0.1 bar to 3.0 bar, preferably 0.3 to 1.5 bar, particularly preferably 0.3 to 0.8 bar; and/or
in particular wherein the mixing elements are arranged in the tubular reactor such that in the operating state, in the first 10 to 30% by volume of the reactor, the conversion of the nitric acid of the nitric acid/sulphuric acid nitrating acid mixture is at least 40%, in particular at least 50%, preferably at least 60%; and/or
in particular wherein the mixing elements are arranged in the tubular reactor such that the conversion of nitric acid introduced is at least 98%, preferably at least 99%, particularly preferably at least 99.5% at the end of the tubular reactor.

11. The production plant according to any one of the preceding claims,
wherein the return device (R) for partially returning the nitrated products to the nitration unit (N) is designed and/or arranged in such a way that the nitrated products to be partially returned are removed from at least one of the following positions (i) to (iv) of the production stream: (i) the crude nitrated aromatic organic compounds (crude nitroaromates), preferably after separation of the acidic aqueous phase (acid phase) and/or after phase separation of the obtained nitration mixture into nitrating acid and crude nitrated aromatic organic compounds (crude nitroaromates); and/or (ii) the washed nitrated aromatic organic compounds (nitroaromates), in particular after acidic or neutral washing; and/or (iii) the washed and stripped or distilled or dried nitrated aromatic organic compounds (nitroaromates), obtained in particular after acidic or neutral washing; and/or (iv) the nitrated aromatic organic compounds (nitroaromates) obtained after the concentration of the nitrating acid in the vapour condensate.

12. The production plant according to any one of the preceding claims,
wherein the return device (R) for partially returning the nitrated products to the nitration unit (N) is designed and/or arranged in such a way that the nitrated products to be partially returned are added and/or supplied to at least one of the following positions (i) to (iv) of the production stream: (i) the initial reaction mixture of all other reactants; and/or (ii) the sulphuric acid of the nitric acid/sulphuric acid nitrating acid mixture, in particular before the preparation of the nitric acid/sulphuric acid nitrating acid mixture; and/or (iii) the nitric acid/sulphuric acid nitrating acid mixture; and/or (iv) the nitrable aromatic organic compounds (aromates) to be nitrated.

13. The production plant according to any one of the preceding claims,
wherein the production plant further comprises at least one recycling device (RA) for recycling the nitrating acid;
in particular wherein the recycling device (RA) comprises a device for concentrating the nitrating acid and optionally a device for adding fresh nitric acid and/or sulphuric acid.

14. A use of nitrated aromatic organic compounds (nitroaromates) to lower the interfacial tension of the organic phase and acid phase and/or to improve the dispersibility of the organic phase and acid phase in nitrations, in particular in nitration reactions of corresponding non-nitrated aromatic organic compounds,
wherein nitrable aromatic organic compounds (aromates) are converted in an adiabatic nitration reaction with a nitric acid/sulphuric acid nitrating acid mixture into the corresponding nitrated aromatic organic compounds (nitroaromates);
wherein corresponding nitrated aromatic organic compounds (nitroaromates) are added to the initial reaction mixture, which comprises the nitrable aromatic organic compounds (aromates) and the nitric acid/sulphuric acid nitrating acid mixture, and the conversion and/or nitration reaction is started and/or carried out in the presence of the nitrated aromatic organic compounds (nitroaromates); and
wherein the nitrated aromatic organic compounds (nitroaromates) obtained are partially returned to the nitration reaction and the subsequent conversion and/or nitration reaction is started and/or carried out in the presence of the nitrated aromatic organic compounds (nitroaromates);
wherein the conversion and/or nitration reaction is carried out under adiabatic reaction conditions,
wherein the conversion and/or nitration reaction is carried out in a tubular reactor, and
wherein the amount of nitrated aromatic organic compounds (nitroaromates) added and/or returned for the conversion and/or nitration reaction is selected such that the amount of nitrated aromatic organic compounds (nitroaromates) added and/or returned reduces the interfacial tension between the organic phase and acid phase and improves dispersibility of the organic phase and acid phase.

15. The use of nitrated aromatic organic compounds (nitroaromates) to increase yields and/or to reduce the formation of by-products and/or to shorten the total reaction times and/or to lower the reaction start temperatures in nitrations, in particular in nitration reactions of corresponding non-nitrated aromatic organic compounds,
wherein nitrable aromatic organic compounds (aromates) are converted in an adiabatic nitration reaction with a nitric acid/sulphuric acid nitrating acid mixture into the corresponding nitrated aromatic organic compounds (nitroaromates);
wherein corresponding nitrated aromatic organic compounds (nitroaromates) are added to the initial reaction mixture, which comprises the nitrable aromatic organic compounds (aromates) and the nitric acid/sulphuric acid nitrating acid mixture, and the conversion and/or nitration reaction is started and/or carried out in the presence of the nitrated aromatic organic compounds (nitroaromates); and
wherein the nitrated aromatic organic compounds (nitroaromates) obtained are partially returned to the nitration reaction and the subsequent conversion and/or nitration reaction is started and/or carried out in the presence of the nitrated aromatic organic compounds (nitroaromates);
wherein the conversion and/or nitration reaction is carried out under adiabatic reaction conditions,
wherein the conversion and/or nitration reaction is carried out in a tubular reactor, and
wherein the amount of nitrated aromatic organic compounds (nitroaromates) added and/or returned for the conversion and/or nitration reaction is selected such that the amount of nitrated aromatic organic compounds (nitroaromates) added and/or returned reduces the interfacial tension between the organic phase and acid phase and improves dispersibility of the organic phase and acid phase.

## Revendications

1. Procédé de nitration adiabatique de composés organiques aromatiques nitrables (aromatiques) en composés organiques aromatiques nitrés correspondants (nitroaromatiques),
dans lequel les composés organiques aromatiques nitrables (aromatiques) sont mis à réagir dans une réaction de nitration avec un mélange sulfonitrique acide nitrique/acide sulfurique pour donner les composés organiques aromatiques nitrés correspondants (nitroaromatiques),
dans lequel les composés organiques aromatiques nitrés correspondants (nitroaromatiques) sont ajoutés au mélange réactionnel de départ, qui comprend les composés organiques aromatiques nitrables (aromatiques) et le mélange sulfonitrique acide nitrique/acide sulfurique, et la conversion et/ou réaction de nitration est démarrée et/ou mise en œuvre en présence des composés organiques aromatiques nitrés (nitroaromatiques); et
dans lequel les composés organiques aromatiques nitrés (nitroaromatiques) obtenus sont partiellement ramenés dans la réaction de nitration et la conversion et/ou réaction de nitration subséquente est démarrée et/ou mise en œuvre en présence des composés organiques aromatiques nitrés (nitroaromatiques);
dans lequel la conversion et/ou réaction de nitration est mise en œuvre dans des conditions de réaction adiabatique,
dans lequel la conversion et/ou réaction de nitration est mise en œuvre dans un réacteur tubulaire et
dans lequel la quantité de composés organiques aromatiques nitrés (nitroaromatiques) ajoutée et/ou ramenée pour la conversion et/ou réaction de nitration est choisie de telle sorte que la quantité de composés organiques aromatiques nitrés (nitroaromatiques) ajoutée et/ou ramenée réduit la tension interfaciale entre la phase organique et la phase acide et améliore la dispersibilité de la phase organique et de la phase acide.

2. Procédé selon la revendication 1,
dans lequel le mélange sulfonitrique final résultant après la conversion et/ou réaction de nitration est recyclé et/ou mis en circulation et/ou ramené à la réaction de nitration après séparation des composés organiques aromatiques nitrés bruts (nitroaromatiques bruts), notamment après concentration et/ou après ajout d'acide nitrique et/ou d'acide sulfurique frais.

3. Procédé selon l'une des revendications précédentes, dans lequel la quantité de composés organiques aromatiques nitrés (nitroaromatiques) ajoutée et/ou ramenée pour la conversion et/ou réaction de nitration est choisie de telle sorte
que la quantité pondérale de composés organiques aromatiques nitrés (nitroaromatiques) ajoutés et/ou ramenés, sur la base des composés organiques aromatiques nitrables (aromatiques) à nitrer et/ou à convertir, se situe dans la plage de 0,01 à 60 % en poids, en particulier dans la plage de 0,1 à 50 % en poids, de préférence dans la plage de 5 à 45 % en poids, de manière particulièrement préférée dans la plage de 10 à 40 % en poids; et/ou
que la quantité pondérale de composés organiques aromatiques nitrés (nitroaromatiques) ajoutés et/ou ramenés, sur la base de l'acide sulfurique du mélange sulfonitrique acide nitrique/acide sulfurique, se situe dans la plage de 0,01 à 10 % en poids, en particulier dans la plage de 0,2 à 5 % en poids, de préférence dans la plage de 0,5 à 3 % en poids, de manière particulièrement préférée dans la plage de 1 à 2 % en poids.

4. Procédé selon l'une des revendications précédentes,
dans lequel les composés organiques aromatiques nitrés (nitroaromatiques) ajoutés et/ou ramenés pour la conversion et/ou réaction de nitration sont ajoutés et/ou amenés à au moins l'une des positions (i) à (iv) suivantes : (i) le mélange réactionnel de départ de tous les autres réactifs ; et / ou (ii) l'acide sulfurique du mélange sulfonitrique acide nitrique/acide sulfurique, notamment avant la préparation du mélange sulfonitrique acide nitrique/acide sulfurique ; et/ou (iii) le mélange sulfonitrique acide nitrique/acide sulfurique ; et/ou (iv) les composés organiques aromatiques nitrables (aromatiques) à nitrer.

5. Procédé selon l'une des revendications précédentes,
dans lequel les composés organiques aromatiques nitrés (nitroaromatiques) ajoutés et/ou ramenés pour la conversion et/ou réaction de nitration proviennent d'au moins l'une des positions (i) à (iv) suivantes : (i) les composés organiques aromatiques nitrés bruts (nitroaromatiques bruts), de préférence après séparation de la phase aqueuse acide (phase acide) et/ou après séparation des phases du mélange de nitration obtenu en mélange sulfonitrique final et composés organiques aromatiques nitrés bruts (nitroaromatiques bruts) ; et/ou (ii) les composés organiques aromatiques nitrés (nitroaromatiques) lavés, notamment après le lavage acide ou neutre ; et/ou (iii) les composés organiques aromatiques nitrés (nitroaromatiques) lavés, notamment obtenus après le lavage acide ou neutre, et également strippés ou distillés ou séchés, et/ou (iv) les composés organiques aromatiques nitrés (nitroaromatiques) se formant dans le condensat de vapeur après la concentration du mélange sulfonitrique final.

6. Procédé selon l'une des revendications précédentes,
dans lequel les composés organiques aromatiques nitrés (nitroaromatiques) ajoutés et/ou ramenés pour la conversion et/ou réaction de nitration sont amenés et/ou ajoutés à la fois à la phase organique et à la phase acide du mélange réactionnel de départ; en particulier dans lequel 0,1 à 35 % en poids, en particulier 10 à 25 % en poids, sur la base de la phase organique, de composés organiques aromatiques nitrés (nitroaromatiques) sont ajoutés à la phase organique, et/ou en particulier dans lequel 0,01 à 3 % en poids, en particulier 0,1 à 2 % en poids, de préférence 0,5 à 1,5 % en poids, de manière particulièrement préférée 1,1 à 1,5 % en poids, sur la base de la phase acide, de composés organiques aromatiques nitrés (nitroaromatiques) sont ajoutés à la phase acide.

7. Procédé selon l'une des revendications précédentes,
dans lequel le mélange sulfonitrique final résultant de la conversion et/ou réaction de nitration est recyclé et/ou mis en circulation et/ou ramené à la réaction de nitration après séparation des composés organiques aromatiques nitrés bruts (nitroaromatiques bruts) et après concentration ultérieure et, le cas échéant, ajout d'acide nitrique et/ou d'acide sulfurique frais, dans lequel les composés organiques aromatiques nitrés (nitroaromatiques) ajoutés et/ou ramenés pour la conversion et/ou réaction de nitration sont ajoutés et/ou amenés au mélange sulfonitrique final concentré et/ou éventuellement complémenté d'acide nitrique et/ou d'acide sulfurique frais et/ou aux composés organiques aromatiques nitrables (aromatiques) à nitrer;
en particulier dans lequel les composés organiques aromatiques nitrables (aromatiques) sont ajoutés et/ou amenés de préférence immédiatement avant le début de la réaction et/ou comme dernier composant réactionnel (réactif), de préférence immédiatement avant la dispersion initiale déclenchant la conversion et/ou réaction de nitration.

8. Procédé selon l'une des revendications précédentes,
dans lequel le mélange sulfonitrique final résultant après la conversion et/ou réaction de nitration est recyclé et/ou mis en circulation et/ou ramené à la réaction de nitration en tant qu'acide de recyclage après séparation des composés organiques aromatiques nitrés bruts (nitroaromatiques bruts) et après concentration ultérieure, dans lequel une dispersion d'acide de recyclage concentré et de composés organiques aromatiques nitrables (aromatiques) à nitrer et de composés organiques aromatiques nitrés (nitroaromatiques) est d'abord produite, dans lequel de l'acide nitrique est ensuite ajouté à la dispersion, en particulier dispersé, et de cette manière, la réaction de nitration est déclenchée;
en particulier dans lequel l'acide nitrique est ajouté et/ou amené de préférence immédiatement avant le début de la réaction et/ou comme dernier composant réactionnel (réactif), de préférence immédiatement avant la dispersion initiale déclenchant la conversion et/ou réaction de nitration.

9. Installation de production (installation de nitration ou installation) pour la nitration adiabatique de composés organiques aromatiques nitrables (aromatiques) en produits nitrés sous forme des composés organiques aromatiques nitrés (nitroaromatiques) correspondants, en particulier installation de production pour la mise en œuvre d'un procédé selon l'une des revendications précédentes,
l'installation de production comprenant les unités et dispositifs suivants :
(a) une unité de nitration (N) pour la nitration, en particulier la nitration adiabatique, des composés organiques aromatiques nitrables (aromatiques) en produits nitrés sous forme des composés organiques aromatiques nitrés (nitroaromatiques) correspondants, en particulier avec un ou plusieurs réacteurs pour mettre en oeuvre la réaction de nitration, l'unité de nitration (N) comprenant au moins un réacteur tubulaire comme réacteur;
(b) disposé dans la ligne de production en aval de l'unité de nitration (N), au moins un dispositif de séparation (S), en particulier un dispositif de séparation (séparateur), pour séparer le mélange sulfonitrique final des produits bruts nitrés;
(c) disposé dans la ligne de production en aval de l'unité de nitration (N) et du dispositif de séparation (S), au moins une unité de lavage (W) pour mettre en œuvre un lavage des produits bruts nitrés avec un milieu de lavage, notamment en une ou plusieurs étapes de lavage;
(d) disposé dans la chaîne de production en aval de l'unité de lavage (W), un dispositif de séparation, notamment un dispositif de séparation (séparateur), pour séparer les produits nitrés lavés du milieu de lavage;
dans lequel l'installation de production comprend également au moins un dispositif de retour (R) pour renvoyer partiellement les produits nitrés vers l'unité de nitration (N), en particulier vers le mélange réactionnel initial de l'unité de nitration (N).

10. Installation de production selon la revendication 9,
dans laquelle le réacteur tubulaire de l'unité de nitration (N) es équipé d'un ou plusieurs, de préférence de plusieurs éléments de mélange, notamment pour introduire une énergie de mélange supplémentaire;
en particulier dans laquelle les éléments de mélange sont conçus comme des tôles, en particulier des tôles de chicane ou de déflexion, comme des panneaux, comme des mélangeurs statiques ou comme des diviseurs de flux; et/ou
en particulier, dans laquelle les éléments de mélange sont conçus de sorte qu'une énergie de mélange de 10 à 1000 joules/litre, de préférence de 10 à 500 joules/litre, de manière particulièrement préférée de 20 à 200 joules/litre, est introduite par les éléments de mélange dans un état de fonctionnement; et/ou
en particulier, dans laquelle dans un état de fonctionnement, la perte de charge par élément de mélange est de 0,1 bar à 3,0 bar, de préférence de 0,3 à 1,5 bar, de manière particulièrement préférée de 0,3 à 0,8 bar; et/ou
en particulier dans laquelle les éléments de mélange sont disposés dans le réacteur tubulaire de telle sorte que dans un état de fonctionnement dans les 10 à 30 premiers % en volume du réacteur, le taux de conversion de l'acide nitrique du mélange sulfonitrique acide nitrique/acide sulfurique est d'au moins 40 %, en particulier d'au moins 50 %, de préférence d'au moins 60 %; et/ou
en particulier dans laquelle les éléments de mélange sont disposés dans le réacteur tubulaire de telle sorte que la transformation de l'acide nitrique utilisé à l'extrémité du réacteur tubulaire est d'au moins 98 %, de préférence d'au moins 99 %, de manière particulièrement préférée d'au moins 99,5 %.

11. Installation de production selon l'une quelconque des revendications précédentes,
dans laquelle le dispositif de retour (R) pour renvoyer partiellement les produits nitrés vers l'unité de nitration (N) est conçu et/ou agencé de telle sorte que les produits nitrés partiellement renvoyés sont prélevés d'au moins l'une des positions (i) à (iv) suivantes du flux de production: (i) les composés organiques aromatiques nitrés bruts (nitroaromatiques bruts), de préférence après séparation de la phase aqueuse acide (phase acide) et/ou après séparation des phases du mélange de nitration obtenu en mélange sulfonitrique final et composés organiques aromatiques nitrés bruts (nitroaromatiques bruts); et/ou (ii) les composés organiques aromatiques nitrés (nitroaromatiques) lavés, notamment après le lavage acide ou neutre ; et/ou (iii) les composés organiques aromatiques nitrés (nitroaromatiques) lavés, notamment obtenus après le lavage acide ou neutre, et également strippés ou distillés ou séchés, et/ou (iv) les composés organiques aromatiques nitrés (nitroaromatiques) se formant dans le condensat de vapeur après la concentration du mélange sulfonitrique final.

12. Installation de production selon l'une quelconque des revendications précédentes,
dans laquelle le dispositif de retour (R) pour renvoyer partiellement les produits nitrés vers l'unité de nitration (N) est conçu et/ou agencé de telle sorte que les produits nitrés partiellement renvoyés sont ajoutés et/ou amenés à au moins l'une des positions (i) à (iv) suivantes du flux de production: (i) le mélange réactionnel de départ de tous les autres réactifs; et/ou (ii) l'acide sulfurique du mélange sulfonitrique acide nitrique/acide sulfurique, notamment avant la préparation du mélange sulfonitrique acide nitrique/acide sulfurique; et/ou (iii) le mélange sulfonitrique acide nitrique/acide sulfurique; et/ou (iv) les composés organiques aromatiques nitrables (aromatiques) à nitrer.

13. Installation de production selon l'une quelconque des revendications précédentes,
dans laquelle l'installation de production comprend en outre au moins un dispositif de recyclage (RA) pour recycler le mélange sulfonitrique final;
en particulier dans laquelle le dispositif de recyclage (RA) comprend un dispositif pour concentrer le mélange sulfonitrique final et éventuellement un dispositif pour ajouter de l'acide nitrique et/ou de l'acide sulfurique frais.

14. Utilisation de composés organiques aromatiques nitrés (nitroaromatiques) pour abaisser la tension interfaciale de la phase organique et de la phase acide et/ou pour améliorer la dispersibilité de la phase organique et de la phase acide lors de nitrations, en particulier dans les réactions de nitration des composés organiques aromatiques non nitrés correspondants,
dans laquelle les composés organiques aromatiques nitrables (aromatiques) sont mis à réagir dans une réaction de nitration adiabatique avec un mélange sulfonitrique acide nitrique/acide sulfurique pour donner les composés organiques aromatiques nitrés (nitroaromatiques) correspondants;
dans lequel les composés organiques aromatiques nitrés correspondants (nitroaromatiques) sont ajoutés au mélange réactionnel de départ, qui comprend les composés organiques aromatiques nitrables (aromatiques) et le mélange sulfonitrique acide nitrique/acide sulfurique, et la conversion et/ou réaction de nitration est démarrée et/ou mise en œuvre en présence des composés organiques aromatiques nitrés (nitroaromatiques); et
dans lequel les composés organiques aromatiques nitrés (nitroaromatiques) obtenus sont partiellement ramenés dans la réaction de nitration et la conversion et/ou réaction de nitration subséquente est démarrée et/ou mise en œuvre en présence des composés organiques aromatiques nitrés (nitroaromatiques);
dans lequel la conversion et/ou réaction de nitration est mise en œuvre dans des conditions de réaction adiabatique,
dans lequel la conversion et/ou réaction de nitration est mise en œuvre dans un réacteur tubulaire et
dans lequel la quantité de composés organiques aromatiques nitrés (nitroaromatiques) ajoutée et/ou ramenée pour la conversion et/ou réaction de nitration est choisie de telle sorte que la quantité de composés organiques aromatiques nitrés (nitroaromatiques) ajoutée et/ou ramenée réduit la tension interfaciale entre la phase organique et la phase acide et améliore la dispersibilité de la phase organique et de la phase acide.

15. Utilisation de composés organiques aromatiques nitrés (nitroaromatiques) pour augmenter les rendements et/ou pour réduire la formation de produits secondaires et/ou pour raccourcir les temps de réaction totaux et/ou pour abaisser les températures de début de réaction lors de nitrations, en particulier dans les réactions de nitration des composés organiques aromatiques non nitrés correspondants,
dans laquelle les composés organiques aromatiques nitrables (aromatiques) sont mis à réagir dans une réaction de nitration adiabatique avec un mélange sulfonitrique acide nitrique/acide sulfurique pour donner les composés organiques aromatiques nitrés (nitroaromatiques) correspondants;
dans lequel les composés organiques aromatiques nitrés correspondants (nitroaromatiques) sont ajoutés au mélange réactionnel de départ, qui comprend les composés organiques aromatiques nitrables (aromatiques) et le mélange sulfonitrique acide nitrique/acide sulfurique, et la conversion et/ou réaction de nitration est démarrée et/ou mise en œuvre en présence des composés organiques aromatiques nitrés (nitroaromatiques); et
dans lequel les composés organiques aromatiques nitrés (nitroaromatiques) obtenus sont partiellement ramenés dans la réaction de nitration et la conversion et/ou réaction de nitration subséquente est démarrée et/ou mise en œuvre en présence des composés organiques aromatiques nitrés (nitroaromatiques);
dans laquelle la conversion et/ou réaction de nitration est mise en œuvre dans des conditions de réaction adiabatique,
dans lequel la conversion et/ou réaction de nitration est mise en œuvre dans un réacteur tubulaire et
dans lequel la quantité de composés organiques aromatiques nitrés (nitroaromatiques) ajoutée et/ou ramenée pour la conversion et/ou réaction de nitration est choisie de telle sorte que la quantité de composés organiques aromatiques nitrés (nitroaromatiques) ajoutée et/ou ramenée réduit la tension interfaciale entre la phase organique et la phase acide et améliore la dispersibilité de la phase organique et de la phase acide.
